# EUROPEAN PATENT APPLICATION

(11) **EP 4 741 506 A1**
(43) Date of publication of application: **13.05.2026**
(21) Application number: 24844743.5
(22) Date of filing: 19.07.2024
(51) Int. Cl.: C12N 15/117, A61K 39/39, A61P 35/00, A61P 37/02

(54) **CPG OLIGODEOXYNUCLEOTIDE CAPABLE OF REGULATING IMMUNE FUNCTION OF BODY AND USE THEREOF**

(30) Priority: 21.07.2023 CN 202310902755
(71) Applicant: Nanjing Jsiama Biopharmaceuticals Ltd., Nanjing, Jiangsu 210000 (CN)
(72) Inventor: LIU, Liming, Nanjing, Jiangsu 210000 (CN); PAN, Qi, Nanjing, Jiangsu 210000 (CN); FU, Yourong, Nanjing, Jiangsu 210000 (CN); MAO, Hui, Nanjing, Jiangsu 210000 (CN)
(74) Representative: Ullrich & Naumann PartG mbB
(86) International application number: PCT/CN2024/106594
(87) International publication number: WO 2025/021052

(57) **Abstract**

The present disclosure discloses a plurality of CpG-containing oligodeoxynucleotides (ODNs) with immunomodulatory properties, and use thereof. The oligodeoxynucleotides provided in present disclosure differ in sequence and structure from the design concept of conventional Type A CpG ODNs, reflecting their own complex drug-forming principles and overcoming the problems of conventional Type A CpG in drug development. The oligodeoxynucleotides exhibit excellent immunomodulatory activity both *in vitro* and *in vivo*, and they can stimulate the production of type I, II, and III interferons, upregulate Th1-type immune responses, and inhibit Th2-type and Th17-type inflammatory responses. Therefore, the oligodeoxynucleotides can: prevent and treat allergic diseases caused by Th2-type immune responses, including allergic rhinitis, atopic dermatitis, asthma, chronic obstructive pulmonary disease, eosinophilia, and Th17-related diseases; can prevent and treat infections and transmission of respiratory or non-respiratory pathogens, including viruses, bacteria, fungi, and parasites; can improve the immune levels of the elderly population and individuals with immunodeficiency; can transform "cold tumors" into "hot tumors" and improve the effectiveness of cancer immunotherapy; can prevent and treat central nervous system diseases related to immune dysfunction, such as Alzheimer's disease; and can be applied to other diseases related to immune dysregulation.

## Description

The present application claims priority of application No. 202310902755.X filed on July 21, 2023 with China National Intellectual Property Administration, entitled "CPG OLIGODEOXYNUCLEOTIDES WITH IMMUNOMODULATORY PROPERTIES AND THE APPLICATIONS THEREOF", the entire content of which is incorporated herein by reference.

### TECHNICAL FIELD

The present disclosure belongs to the field of oligodeoxynucleotides, specifically relating to a CpG oligodeoxynucleotides with immunomodulatory properties and the applications thereof.

### BACKGROUND OF THE INVENTION

TLR9 is an innate immune receptor in mammals, located in endosomes. Its ligands are oligodeoxynucleotides (ODNs) containing unmethylated Cytosine-Phosphorothioate-Guanine (CpG). Depending on their type and structure, CpG-ODNs, after entering the endosome, can bind to TLR9 and activate the IRF-7 or NF-κB signaling pathways, producing inflammatory factors such as type I interferons or IL-6 to intervene in the immune response.

Based on structural characteristics and immune effects, CpG ODNs can be classified into three classes: A, B, and C. Type A CpG enters the early endosomes of plasmacytoid dendritic cells (pDCs), binds to the TLR9 receptor therein, activates the IRF-7 signaling pathway, and induces the production of large amounts of type I interferons (IFN-α/IFN-β) and type III interferon (IFN-λ). Type I interferons, as a general immunomodulatory cytokine, acts on downstream immune cells, including but not limited to NK cells, CD8⁺ T cells, CD4⁺ T cells, γ-δ T cells, and macrophages, inducing the production of cytokines such as type II interferon (IFN-γ). Type B CpG is a fully thiolated linear CpG ODN, primarily targeting B lymphocytes. Type B CpG enters late endosomes and binds to TLR9, activating NF-κB and generating a series of immune responses, including the secretion of inflammatory cytokines such as IL-6, IL-1, and TNF-α, and promoting B cell proliferation. Type B CpG can be used as a vaccine adjuvant, significantly increasing antibody levels. Class C CpG is a fully thiolated CpG ODN, containing partial structures of Type A CpG and Type B CpG. It possesses both palindromic sequences capable of forming dimers and non-palindromic linear structures. Class C CpG can enter both early and late endosomes and bind to TLR9. Therefore, Class C CPG possesses the activity of both Type A and Type B CpG-ODNs, activating both the IRF-7 signaling pathway and the NF-κB pathway to produce type I interferons and inflammatory cytokines. While Class C CPG combines the characteristics of both Type A and Type B CpGs, its ability to induce interferons and inflammatory molecules is significantly lower than that of either Type A or Type B CpGs individually.

Preclinical animal studies of Type B CpG have demonstrated its successful treatment of allergic rhinitis, asthma, various microbial infections, and anti-tumor effects. Over the past 20 years, more than 100 publications have reported on the preclinical treatment of various diseases with CpGs. CPG-1018, a representative Type B CpG, was successfully approved for marketing as an adjuvant for hepatitis B vaccines in 2017.

Current research indicates that when administered via different routes, Type A CpG ODN can induce robust innate immunity (type I/II/III interferons) and Th1 immune responses (activation of DCs, NK and CD8⁺ T cells, and IL-12 production) by targeting TLR9 in pDCs, and can also modulate immune function, thereby inhibiting Th2 and Th17 immune responses. These immune responses can be used to treat diseases caused by allergic and non-allergic inflammation, combat pathogenic microbial infections, directly or indirectly kill tumor cells, reduce Aβ plaque formation, and enhance vaccine immunogenicity, among other effects. This also laid the foundation for its application in infectious diseases, cancer, immunoadjuvants, allergic rhinitis, atopic dermatitis, asthma, chronic obstructive pulmonary disease, eosinophilic disease, Alzheimer's disease, wound healing, and wound infection prevention and control (Montamat et al., Fronteers in Immunol. 2021. doi: 10.3389/fimmu.2021.590054; Scheiermann et al., Vaccine 2014. 32:6377-6389; Shirota and Klinman., Immunopotentiators in Modern Vaccines ISBN 978-0-12-804019-5, http://dx.doi.org/10.1016/B978-0-12-804019-5.00009-8; Beeh et al., J Allergy Clin Immunol 2013;131:866-74; Hanagata N. et al., Int J Nanomedicine. 2012;7:2181-95; Kayraklioglu et al., Methods Mol Biol. 2021;2197:51-85; Fan et al., Clin Cancer Res. 2012 Oct 15;18(20):5628-38; Senti et al., Clin Exp Allergy. 2009 Apr;39(4):562-70; Scholtzova et al., Acta Neuropathol Commun. 2014 Sep 2;2:101; and Wanke-Jellinek et al., J Immunol. 2016 Jan 15;196(2):767-77).

Due to the tendency of Type A CpG to multimerize, its drugability is very poor. As a result, more than 20 years after its discovery, there have been very few clinical trials for Type A CpG, and no drugs have been approved for marketing. Historically, three institutions have attempted to improve the drugability of Type A CpG through various methods. 1. Cytos Biotechnology AG encapsulated Type A CpG with virus-like particles (VLPs) (QbG-10). While this improved drugability, the VLP encapsulation transformed Type A CpG from a simple CPG into a CPG with strong adjuvant function (Stomi et al., J. Immunol. 2004. 172:1777-1785). 2. Scientists at the FDA added fma (a small molecule compound) to the five Gs at the 3' end of Type A CpG, making it a temperature-sensitive prodrug. After entering cells, the fma automatically detaches at 37°C, causing Type A CpG to re-multimerize and exert its therapeutic effect. However, such modification cannot completely prevent multimerization *in vitro,* and the modification of fma limits the drug's safety and tolerability (Puig et al., Puig, Nucleic acids research. 2005. 34:6488-6495). 3. In 2015, Japanese scientists reported that adding 40 adenine deoxyribonucleotides (polyAs) to the 3' end of Type A CpG significantly improved its drugability. However, the addition of 40 adenine nucleotides greatly increased production costs, and because it is fully thiolated, it can increase the production of the IL-6 inflammatory molecule, thus limiting its safety and tolerability (Aoshi et al., J. Immunol. Research. 2015. http://dx.doi.org/10.1155/2015/316364). Therefore, there are currently no successful cases of conventional Type A CpG being developed into drugs, and no clinical trials have been reported.

The conventional design principle of Type A CpG involves generating multimers through a complete palindrome of a core sequence having 8-12 nucleotides (including one or more CpGs) and poly-Gs at both ends. These multimers present the CpG core sequence to TLR9 in early endosomes rather than late endosomes, thereby activating the IRF-7 signaling pathway and producing type I interferons. Multimerization is essential for IRF-7 activation (Kerkmann et al., J. Bio. Chem., 2005. 280:8086-8093; and Wu et al., J. Bio. Chem., 2004. 279:33071-33078). Monomeric CPG ODNs, however, cannot activate the IRF-7 signaling pathway. For example, Type B CPG, being a monomer in solution, can only bind to TLR9 in late endosomes, thereby activating the NF-κB signaling pathway and inducing the production of inflammatory factors rather than type I interferons. Type A CpGs must form multimers to be active, but the formation of these multimers often results in large, uncontrollable clusters, significantly reducing their drugability. This is the main reason why there have been no successful clinical development cases of Type A CpGs since they were discovered. Therefore, ensuring that Type A CpGs exhibit multimerization while maintaining controllable multimerization is crucial for developing these molecules into clinically effective drugs.

The conventional self-multimerization model of Type A CpGs was proposed by Kerkmann *et al.* in 2005 (Kerkmann et al., J. Bio. Chem., 2005. 280:8086-8093): Two palindromic complementary monomers are linked by Watson-Crick to form a dimer. The poly-Gs at both ends of the dimer form a Hoogsteen base pair through hydrogen bonds of guanosine, becoming a tetramer (G-tetrad). The dimer and tetramer then continue to multimerize through G-tetrads, cycling to form even larger multimers. Another model is based on the secondary structure of Type A CpG. Type A CpG monomers can form a palindromic structure within the monomer, and the poly-Gs at both ends can pair with each other through Hoogsteen to form larger multimers. Therefore, from these models, we can see that the size and stability of the multimers depend on the number of surrounding Hoogsteen connections and their unique spatial structure. Our earlier research found that, from a secondary structure perspective, a completely palindromic form before and after the poly-G can reduce the energy required for multimerization, making multimerization easier. Therefore, establishing a certain spatial hinderance between the poly-G and the palindrome may increase the energy required to form G-tetrads, making the multimers relatively difficult to form or relatively loose after formation. At the same time, combining the selection of the number of poly-G at the two ends may stabilize the multimers within a certain size range. Based on the above considerations, multiple core sequences were designed. Meanwhile, while keeping the core sequences unchanged, deoxyribonucleotide substitution experiments were performed on the two ends of Type A CPG to find out the formation pattern of Type A CPG multimers and the relationship thereof with stimulation of type I interferon production.

### SUMMARY OF THE INVENTION

The technical solutions of the present disclosure are as below:

As provided in this invention is a CpG oligodeoxynucleotide with immunomodulatory properties, which comprises a sequence having the structure of 5'-(G)ₙ-core sequence-(N)_{q}-(G)m-3', wherein the core sequence is a palindromic sequence, N is any one of adenine deoxyribonucleotide (A), thymine deoxyribonucleotide (T), and cytosine deoxyribonucleotide (C); G is guanine deoxyribonucleotide; q, n, and m are all integers, in which n ranges from 2 to 11, q ranges from 0 to 10, and m ranges from 2 to 11.

Preferably, n ranges from 3 to 4, and q ranges from 1 to 5.

Preferably, the core sequence is at least one selected from the group consisting of CGCGACGCGTCGCG, ACGATCGAGATCGT, AGGATCGATCCT, TTCGATCGATCGAA, CGATCGATCG, GACGATCGTC, and TGCATCGATGCA; wherein
if the core sequence is CGCGACGCGTCGCG, q ranges from 0 to 10, n ranges from 0 to 3, and m ranges from 2 to 11;
or if the core sequence is ACGATCGAGATCGT, q ranges from 0 to 10, n ranges from 2 to 11, and m ranges from 2 to 11;
or if the core sequence is AGGATCGATCCT, q ranges from 0 to 10, n ranges from 2 to 11, and m ranges from 2 to 11;
or if the core sequence is TTCGATCGATCGAA, q ranges from 0 to 10, n ranges from 2 to 11, and m ranges from 2 to 11;
or if the core sequence is CGATCGATCG, q ranges from 0 to 10, n ranges from 1 to 11, and m ranges from 2 to 11;
or if the core sequence is GACGATCGTC, then q is 0, n ranges from 0 to 3, and m ranges from 2 to 11, or then q ranges from 1 to 10, n ranges from 2 to 11, and m ranges from 2 to 11;
or if the core sequence is TGCATCGATGCA, then q is 0, n has a value of 1, 3, 4 or 5, and m ranges from 2 to 11, or then q ranges from 1 to 10, n ranges from 2 to 11, and m ranges from 2 to 11.

Preferably, the CpG oligodeoxynucleotide as described above comprises at least one deoxynucleotide of SEQ ID NO.1 - SEQ ID NO.82.

Preferably, the CpG oligodeoxynucleotide may comprise at least one selected from the group consisting of: thio-modification, fluorination-modification, methoxy-modification, locked nucleic acid modification, or nanoparticle modification.

Preferably, the thio-modification occurs at the 5' and/or 3' ends of polyguanosine (poly-G).

Preferably, the nanoparticles used in the nanoparticle modification include at least one selected from group consisting of PLGA, chitosan, lipid nanoparticles, and liposomes.

A CpG oligodeoxynucleotide solution formulation, comprising the CpG oligodeoxynucleotide as described above and excipients, wherein the excipients comprise at least one selected from the group consisting of: pH buffer pair, glycine, trehalose, mannitol, sucrose, arginine, lysine, histidine, glycerol, propylene glycol, Pluronic F127, Pluronic F68, Tween 20, Tween 80, benzalkonium chloride, disodium edetate, sodium citrate, hydroxypropyl methylcellulose, sodium carboxymethyl cellulose, methyl betacyclodextrin, and polyethylene glycol.

Preferably, metal ions in the CpG oligodeoxynucleotide formulation include sodium, potassium, magnesium, calcium, zinc, and iron, with a total concentration ranging from 0.1 to 90 mM.

Preferably, the pH of the CpG oligodeoxynucleotide formulation ranges from 7 to 9.

Use of the CpG oligodeoxynucleotide as described above in preparation of drugs for regulating respiratory immune responses.

Use of the CpG oligodeoxynucleotide as described above in preparation of drugs for prevention and treatment of respiratory diseases, including at least one selected from the group consisting of respiratory viral infections, respiratory bacterial infections, respiratory fungal infections, respiratory parasitic infections, and respiratory allergic diseases. The respiratory viruses involved in the respiratory viral infections include at least one selected from the group consisting of: COVID-19 virus, influenza virus, RSV virus, and SARS-CoV.

Use of the CpG oligodeoxynucleotide as described above in preparation of drugs for prevention and treatment of non-respiratory tract infections, including at least one selected from the group consisting of HIV infection, HBV infection, and HCV infection.

Use of the CpG oligodeoxynucleotide as described above in preparation of drugs for regulating proliferation of immune cells or release of immune cell cytokines.

Use of the CpG oligodeoxynucleotide as described above in preparation of immunoadjuvants.

Use of the CpG oligodeoxynucleotide as described above in preparation of drugs for prevention and treatment of viral infections.

Use of the CpG oligodeoxynucleotide as described above in preparation of drugs for treatment of tumors, wherein the CpG oligodeoxynucleotide is the only anti-tumor active ingredient.

An anti-tumor composition, comprising the CpG oligodeoxynucleotide as described above and an anti-tumor drug, wherein the anti-tumor drug includes, but is not limited to, PD-1 anti-tumor drugs, PDL-1 anti-tumor drugs and anti-tumor cell therapy, wherein the CpG oligodeoxynucleotide in the composition serves as a component with anti-tumor efficacy or as an immunoadjuvant.

Use of the CpG oligodeoxynucleotide as described above in preparation of human or animal vaccines.

Use of the CpG oligodeoxynucleotide as described above in treatment of central nervous system diseases, wherein the central nervous system diseases include, but are not limited to, Alzheimer's disease.

Use of the CpG oligodeoxynucleotide as described above in preparation of drugs for prevention and treatment of secondary infections after trauma.

Use of the CpG oligodeoxynucleotide as described above in preparation of drugs for promoting wound healing.

Use of the CpG oligodeoxynucleotide as described above in preparation of drugs for treatment of allergic rhinitis and/or atopic dermatitis and/or asthma and/or chronic obstructive pulmonary diseases and/or eosinophilic-related diseases.

A drug prepared using the CpG oligodeoxynucleotide as described above, wherein the drug is in a form of an injection, tablet, lyophilized preparation, inhalant, nasal drops, nasal spray, rectal suppository, eye drops, ointment, lotion, or gel.

A CpG oligodeoxynucleotide topical formulation, comprising the CpG oligodeoxynucleotide of any one of claims 1 to 5 and excipients, wherein the excipients include glyceryl behenate, liquid paraffin, petrolatum, medium-chain triglycerides, cocoyl octanoate/decanoate, glyceryl mono- and distearate, hydroxypropyl methylcellulose, carbomer, xanthan gum, sodium carboxymethyl cellulose, poloxamer 407, polyethylene glycol cetostearyl ether, poloxamer 188, oleoyl polyoxylglycerides, PEG-7 stearate, Labrasol, sodium lauryl sulfate, propylene glycol monofatty acid ester, polyglyceryl oleate, propylene glycol laurate, propylene glycol, isopropyl myristate, and polyglyceryl oleate.

A formulation of the CpG oligodeoxynucleotide adjuvant as described above itself or in combination with other adjuvants for use in human or animal vaccines, comprising the CpG oligodeoxynucleotide itself, or a combination thereof with other adjuvants, said other adjuvants are preferably aluminum hydroxide adjuvant and aluminum phosphate adjuvant.

The beneficial effects of the present disclosure are as follows:

Unlike the conventional palindromic design of Type A CpG, the present disclosure provides a novel CpG ODN sequence with a novel structure and sequence. By replacing the nucleotides at both ends of the ODN of those sequences, the relationship and related patterns between their drugability and activity were identified. Compared to published sequences, they possess unique primary and secondary structures, overcoming the problem of poor drugability and easy multimerization of conventional Type A CpGs, and thus showing higher immunomodulatory activity and drugability. In particular, the AM1012-05 sequence, whose secondary structure is more stable at higher temperatures than similar products, demonstrates better multimerization controllability than other similar sequences, and exhibits better batch-to-batch stability and drugability during production procedure. Meanwhile, AM1012-05 can effectively penetrate the human respiratory tract epithelial mucosa (RPMI 2650 cells) in a penetration model and exhibits excellent immune-activating activity in animals. It demonstrates excellent ability to block respiratory viral infection and transmission, and has the potential, by activating and regulating the immune system, to exert anti-tumor effects, treat allergic rhinitis, asthma, chronic obstructive pulmonary disease, eosinophilic disease, atopic dermatitis and Alzheimer's disease, and be used for prevention and treatment of secondary infection after wound, and promote wound healing. It also has the potential to be used as an independent adjuvant or component of an adjuvant system in vaccines.

### BRIEF DESCRIPTION OF THE DRAWINGS

To more clearly illustrate the technical solution of the present disclosure, the drawings used in the embodiments will be briefly introduced below. Apparently, for those skilled in the art, other drawings can be obtained based on these drawings without creative effort.
Figure 1 shows the evaluation of the IFN-γ stimulatory effects by different CpG sequences on NK-92 cells, in which Panels A, B, C, D, and E show the detected IFN-γ stimulatory effects by different CpG sequences on NK-92 cells under the same experimental system;
Figure 2 shows the comparison of stimulatory effects of different CpG sequences on SD rat spleen cells, in which Panel A shows the IFN-γ secretion of rat spleen cells stimulated by different CpG sequences, Panel B shows the TNF-α secretion of rat spleen cells stimulated by different CpG sequences, and Panel C shows the IL-6 secretion of rat spleen cells stimulated by different CpG sequences;
Figure 3 shows the evaluation of the IFN-γ stimulatory effects by AM1012-05 on NK-92 cells;
Figure 4 shows the evaluation of the stimulatory effects by AM1012-05 on BALB/c mouse spleen cells, in which Panel A shows the IL-6 secretion of BALB/c mouse spleen cells stimulated by AM1012-05, Panel B shows the IFN-γ secretion of BALB/c mouse spleen cells stimulated by AM1012-05, Panel C shows the TNF-α secretion of BALB/c mouse spleen cells stimulated by AM1012-05, and Panel D shows the TNF-α secretion of BALB/c mouse spleen cells AM1012-05 stimulated by AM1012-05;
Figure 5 shows the evaluation of the stimulatory effects by AM1012-05 on C57BL6 mouse spleen cells, in which Panel A shows the IL-6 secretion of C57BL6 mouse spleen cells stimulated by AM1012-05, Panel B shows the IFN-γ secretion of C57BL6 mouse spleen cells stimulated by AM1012-05, Panel C shows the TNF-α secretion of C57BL6 mouse spleen cells stimulated by AM1012-05, and Panel D shows the IFN-α secretion of C57BL6 mouse spleen cells AM1012-05 stimulated by AM1012-05;
Figure 6 shows the evaluation of the stimulatory effect of AM1012-05 on cotton rat spleen cells, in which Panel A shows that AM1012-05 increases the mRNA level of IFN-α in cotton rat spleen cells, Panel B shows that AM1012-05 increases the mRNA level of IFN-γ in cotton rat spleen cells, Panel C shows that AM1012-05 increases the mRNA level of TNF-α in cotton rat spleen cells, and Panel D shows that AM1012-05 increases the mRNA level of CXCL-10 in cotton rat spleen cells;
Figure 7 shows the evaluation of the stimulatory effect (mRNA level) of AM1012-05 on rat spleen cells, in which Panel A shows that AM1012-05 increases the mRNA level of IFN-α in rat spleen cells, and Panel B shows that AM1012-05 increases the mRNA level of CXCL-10 in rat spleen cells;
Figure 8 shows the evaluation of the stimulatory effects (protein level) of AM1012-05 on rat spleen cells, in which Panel A shows the IFN-γ secretion of rat spleen cells stimulated by AM1012-05, Panel B shows the IL-6 secretion of rat spleen cells stimulated by AM1012-05, and Panel C shows the TNF-α secretion of rat spleen cells stimulated by AM1012-05;

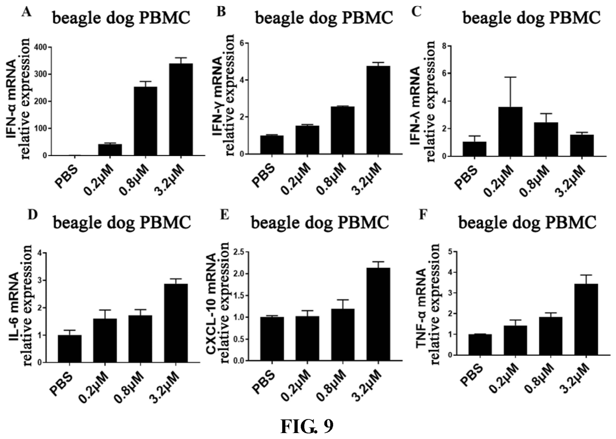

Figure 9 shows the evaluation of the stimulatory effect of AM1012-05 on beagle dog PBMCs, in which Panel A shows that AM1012-05 increases the mRNA level of IFN-α in beagle dog PBMCs, Panel B shows that AM1012-05 increases the mRNA level of IFN-γ in beagle dog PBMCs, Panel C shows that AM1012-05 increases the mRNA level of IFN-λ in beagle dog PBMCs, Panel D shows that AM1012-05 increases the mRNA level of IL-6 in beagle dog PBMCs, Panel E shows that AM1012-05 increases the mRNA level of CXCL-10 in beagle dog PBMCs, and Panel F shows that AM1012-05 increases the mRNA level of TNF-α in beagle dog PBMCs;

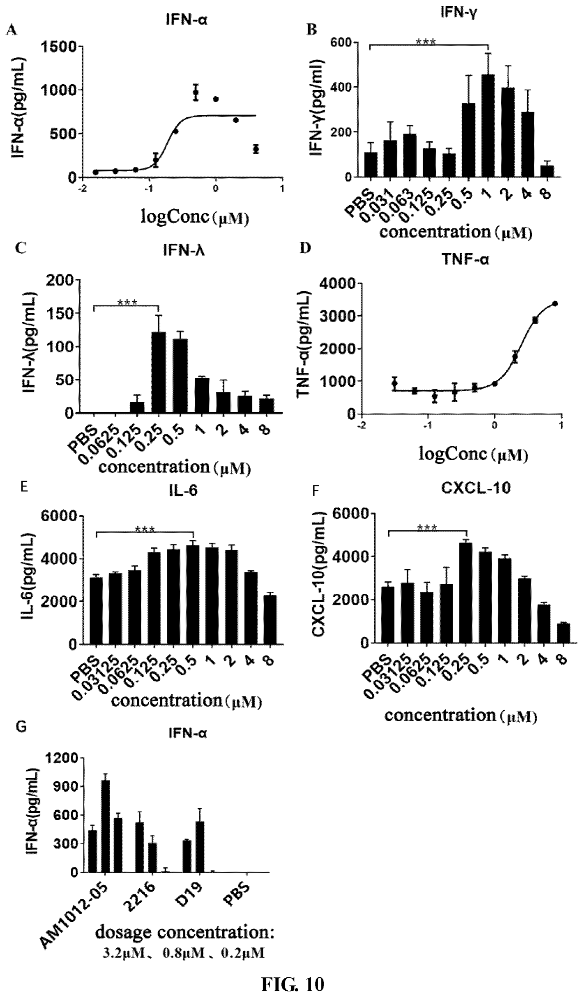

Figure 10 shows the evaluation of the stimulatory effect of CpG ODN on human PBMCs, in which Panel A shows the IFN-α secretion of human PBMCs stimulated by AM1012-05, Panel B shows the IFN-γ secretion of human PBMCs stimulated by AM1012-05, Panel C shows the IFN-λ secretion of human PBMCs stimulated by AM1012-05, Panel D shows the TNF-α secretion of human PBMCs stimulated by AM1012-05, Panel E shows the IL-6 secretion of human PBMCs stimulated by AM1012-05, Panel F shows the CXCL-10 secretion of human PBMCs stimulated by AM1012-05, and Panel G shows the IFN-α secretion of human PBMCs stimulated by AM1012-05, CpG 2216 and CpG D19;
Figure 11 shows the electrophoresis detection results of serum stability of CpG ODN;
Figure 12 shows the multimer distribution of different CpG ODN multimers; in which the left Panel shows the electrophoretic detection of distribution of Type A CpG multimers (2 hours), and the right Panel shows the electrophoretic detection of distribution of Type A CpG multimers (24 hours);
Figure 13 shows the multimer analysis of AM1012-05 and CpG 2216 solutions in static and vortex states;
Figure 14 shows the comparison of secondary structures of different sequence samples at - 20°C, in which Panel A shows CpG 2216, Panel B shows E41, Panel C shows AM1012-03, and Panel D shows AM1012-05;
Figure 15 shows the comparison of secondary structures of different sequence samples at 0°C, in which Panel A shows CpG 2216, Panel B shows E41, Panel C shows AM1012-03, and Panel D shows AM1012-05;
Figure 16 shows the comparison of secondary structures of different sequence samples at 4°C, in which Panel A shows CpG 2216, Panel B shows E41, Panel C shows AM1012-03, and Panel D shows AM1012-05;
Figure 17 shows the comparison of secondary structures of different sequence samples at 25°C, in which Panel A shows CpG 2216, Panel B shows E41, Panel C shows AM1012-03, and Panel D shows AM1012-05;
Figure 18 shows the comparison of secondary structures of different sequence samples at 37°C, in which Panel A shows CpG 2216, Panel B shows E41, Panel C shows AM1012-03, and Panel D shows AM1012-05;
Figure 19 shows the comparison of secondary structures of different sequence samples at 40°C, in which Panel A shows CpG 2216, Panel B shows E41, Panel C shows AM1012-03, and Panel D shows AM1012-05;
Figure 20 shows the comparison of secondary structures of different sequence samples at 50°C, in which Panel A shows CpG 2216, Panel B shows E41, Panel C shows AM1012-03, and Panel D shows AM1012-05;
Figure 21 shows the comparison of secondary structures of different sequence samples at 60°C, in which Panel A shows CpG 2216, Panel B shows E41, Panel C shows AM1012-03, and Panel D shows AM1012-05;
Figure 22 shows the comparison of secondary structures of different sequence samples at 70°C, in which Panel A shows CpG 2216, Panel B shows E41, Panel C shows AM1012-03, and Panel D shows AM1012-05;
Figure 23 shows the comparison of secondary structures of different sequence samples at 80°C, in which Panel A shows CpG 2216, Panel B shows E41, Panel C shows AM1012-03, and Panel D shows AM1012-05;
Figure 24 shows the comparison of secondary structures of different sequence samples at 90°C, in which Panel A shows CpG 2216, Panel B shows E41, Panel C shows AM1012-03, and Panel D shows AM1012-05;
Figure 25 shows the effect of CpG ODN treated at different temperatures on IFN-γ secretion in rat spleen cells;
Figure 26 shows the particle size analysis of PLGA nanoparticles of CpG ODNs;
Figure 27 shows the particle size analysis of chitosan nanoparticles of CpG ODNs;
Figure 28 shows the particle size analysis of lipid nanoparticles of CpG ODNs;
Figure 29 shows the particle size analysis of prepared PLGA nanoparticles of CpG ODNs as an immunoadjuvant;
Figure 30 shows the effect of formulation components on the IFN-γ stimulatory effects of AM1012-05 on NK-92 cells;

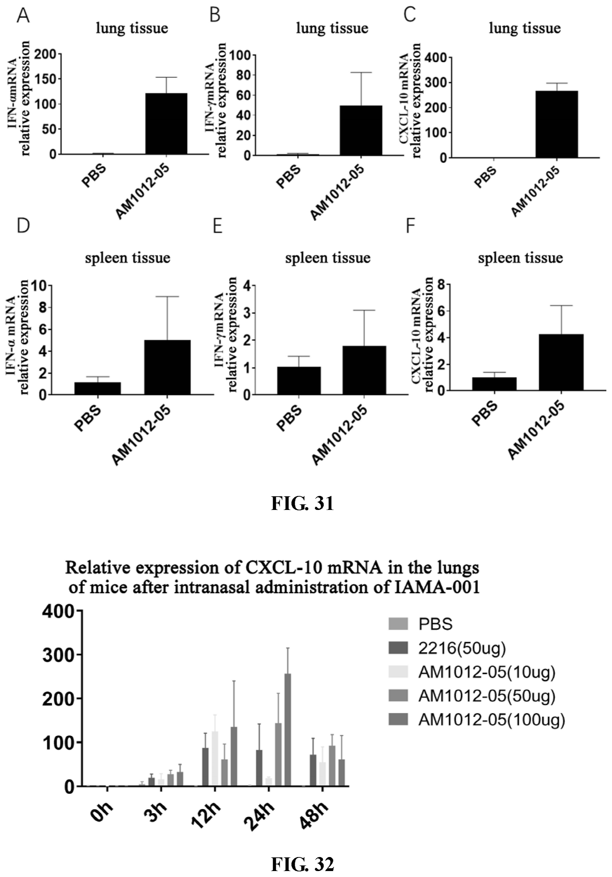

Figure 31 shows the effect of AM1012-05 on the relative expression levels of immune-related mRNAs in mouse lung and spleen tissues, in which Panel A shows that AM1012-05 increases the mRNA level of IFN-α in mouse lung tissue, Panel B shows that AM1012-05 increases the mRNA level of IFN-γ in mouse lung tissue, Panel C shows that AM1012-05 increases the mRNA level of CXCL-10 in mouse lung tissue, Panel D shows that AM1012-05 increases the mRNA level of IFN-α in mouse spleen tissue, Panel E shows that AM1012-05 increases the mRNA level of IFN-γ in mouse spleen tissue, and Panel F shows that AM1012-05 increases the mRNA level of CXCL-10 in mouse spleen tissue;
Figure 32 shows the relative expression level of CXCL-10 mRNA in mouse lung after intranasal administration of AM1012-05;
Figure 33 shows the cytokine secretion induced by AM1012-05 in mouse lung tissue at different dose frequencies, in which Panel A shows the CXCL-10 secretion induced by AM1012-05 in mouse lung tissue at different dose frequencies, and Panel B shows the IFN-γ secretion induced by AM1012-05 in mouse lung tissue at different dose frequencies;
Figure 34 shows the killing effect of AM1012-05-activated human PBMCs on N87 cells;
Figure 35 shows the killing effect of AM1012-05-activated human PBMCs on MDA-MB-231 cells;

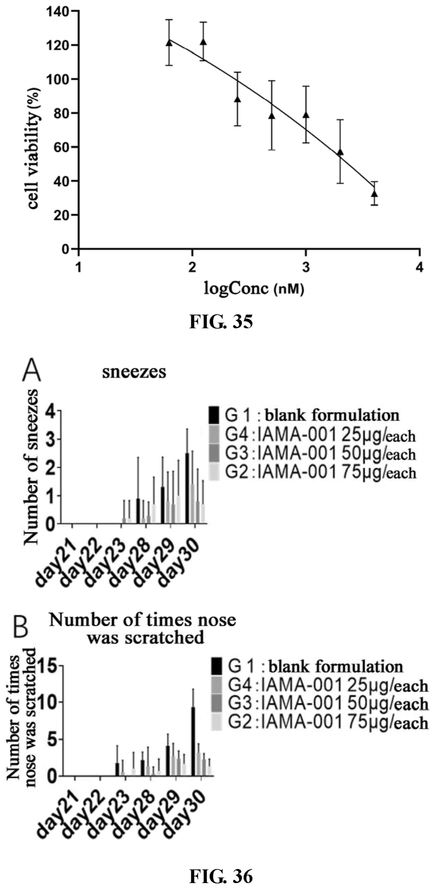

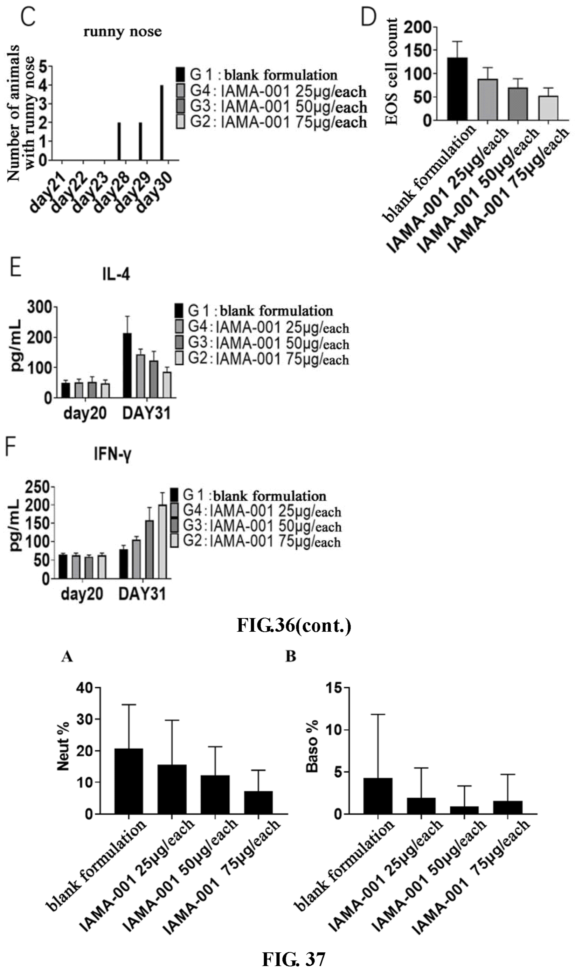

Figure 36 shows the ability of AM1012-05 to alleviate allergic rhinitis in mice, in which Panel A shows that AM1012-05 reduces the number of sneezes in mice with allergic rhinitis, Panel B shows that AM1012-05 reduces the number of nose-scratching in mice with allergic rhinitis, Panel C shows that AM1012-05 reduces runny nose in mice with allergic rhinitis, Panel D shows that AM1012-05 reduces the number of eosinophils in the nasal mucosa of mice with allergic rhinitis, Panel E shows that AM1012-05 reduces the expression of IL-4 in the serum of mice with allergic rhinitis, and Panel F shows that AM1012-05 increases the expression of IFN-γ in the serum of mice with allergic rhinitis;
Figure 37 shows the results of immune cell analysis of nasal lavage fluid from mice treated with AM1012-05 for allergic rhinitis;
Figure 38 shows the in vitro anti-respiratory syncytial virus (RSV) infection activity of AM1012-05, in which Panel A shows the prevention of RSVs proliferation in cells achieved by co-incubation of AM1012-05 with PBMCs, and Panel B shows the clearance of RSVs from host cells by AM1012-05;
Figure 39 shows the anti-atopic dermatitis activity of AM1012-05 (IgE level);
Figure 40 shows the adjuvant application of AM1012-05, in which Panel A shows the titers of IgG 7 days after the first immunization; Panel B shows the titers of IgG 7 days after the second immunization; Panel C shows the titers of IgG 7 days after the third immunization; Panel D shows the titers of IgG 21 days after the third immunization; Panel E shows the titers of IgG 35 days after the third immunization;
Figure 41 shows the adjuvant application of AM1012-05, in which Panel A shows the titers of IgG1 7 days after the first immunization; Panel B shows the titers of IgG1 7 days after the second immunization; Panel C shows the titers of IgG1 7 days after the third immunization; Panel D shows the titers of IgG1 21 days after the third immunization; Panel E shows the titers of IgG1 35 days after the third immunization; and
Figure 42 shows the adjuvant application of AM1012-05, in which Panel A shows the titers of IgG2a 7 days after the first immunization; Panel B shows the titers of IgG2a 7 days after the second immunization; Panel C shows the titers of IgG2a 7 days after the third immunization; Panel D shows the titers of IgG2a 21 days after the third immunization; Panel E shows the titers of IgG2a 35 days after the third immunization.

### DETAILED DESCRIPTION OF THE INVENTION

The specific embodiments of the present disclosure are described in detail below. Obviously, the described embodiments are only a part, and not all, of the embodiments of the present disclosure. All other embodiments obtained by those skilled in the art based on the embodiments of the present disclosure without creative effort are within the scope of protection of the present disclosure.

### Example 1. Design and Synthesis of CpG ODN Molecules

Multiple CpG ODN sequences were designed in the present disclosure (see Table 1) and synthesized by solid-phase synthesis method. The final product was then obtained through ammonolysis, purification, desalting, concentration, dispensing and lyophilization. The specific processes are as follows:
(1) Solid-phase synthesis: a computer-controlled solid-phase synthesis, consisting of four repetitive synthesis steps (detritylation, coupling, sulfidation, and capping) and a final step of removing the protecting group from the phosphoric acid backbone;
(2) Ammonolysis: After synthesis, ammonia or organic amines were used to cleave the oligonucleotides from the substrate to be dissolved into a solution, and the universal linker group connected to the first base at the 3' end and the protecting group on the amino group were removed;
(3) Purification: The crude product obtained after ammonolysis was purified and separated using ion exchange chromatography, reversed-phase chromatography, or electrophoresis to obtain a purified product;
(4) Desalting and concentration: The purified product was desalted and concentrated using ultrafiltration equipment; and
(5) Dispensing and lyophilization: Lyophilization yielded a white, off-white or pale yellow loose product. Nucleic acid modifications include, but are not limited to, thio-modification.

**Table 1. Sequences involved in the present disclosure**

| # | ID | Sequence |
|---|---|---|
| 1 | AM1012-01 | G*G*CGCGACGCGTCGCG*G*G*G*G*G |
| 2 | AM1012-02 | G*G*ACGATCGAGATCGTG*G*G*G*G*G |
| 3 | AM1012-03 | G*G*AGGATCGATCCTG*G*G*G*G*G |
| 4 | AM1012-04 | G*G*TTCGATCGATCGAAG*G*G*G*G*G |
| 5 | AM1012-05 | G*G*GGCGATCGATCGAAG*G*G*G*G*G |
| 6 | AM1012-06 | GGGGCGATCGATCGAAGGGGGG |
| 7 | AM1012-07 | G*G*GCGCGACGCGTCGCG*G*G*G*G*G |
| 8 | AM1012-37 | G*GGCGATCGATCGAAG*G*G*G*G*G |
| 9 | AM1012-38 | G*G*GGCGATCGATCGAAGG*G*G*G*G |
| 10 | AM1012-39 | GGCGATCGATCGAAG*G*G*G*G*G |
| 11 | AM1012-40 | GCGATCGATCGAAG*G*G*G*G*G |
| 12 | AM1012-30 | G*G*AGGATCGATCCT*G*G |
| 13 | AM1012-31 | G*G*G*AGGATCGATCCT*G*G*G |
| 14 | AM1012-32 | G*G*G*G*AGGATCGATCCT*G*G*G*G |
| 15 | AM1012-16 | G*G*G*G*GAGGATCGATCCTG*G*G*G*G |
| 16 | AM1012-17 | G*G*G*G*G*GAGGATCGATCCTG*G*G*G*G*G |
| 17 | AM1012-18 | G*G*G*G*G*G*GAGGATCGATCCTG*G*G*G*G*G*G |
| 18 | AM1012-19 | G*G*G*G*G*G*G*GAGGATCGATCCTG*G*G*G*G*G*G*G |
| 19 | AM1012-20 | G*G*G*G*G*G*G*G*GAGGATCGATCCTG*G*G*G*G*G*G*G*G |
| 20 | AM1012-21 | G*G*G*G*G*G*G*G*G*GAGGATCGATCCTG*G*G*G*G*G*G*G*G*G |
| 21 | AM1012-22 | G*G*G*G*G*G*G*G*G*G*GAGGATCGATCCTG*G*G*G*G*G*G*G*G*G*G |
| 22 | AM1012-13 | GGGGAGGATCGATCCTGGGGGG |
| 23 | AM1012-14 | GGGGGGAGGATCGATCCTGGGGGG |
| 24 | AM1012-33 | G*G*CGATCGATCGAA*G*G |
| 25 | AM1012-34 | G*G*G*CGATCGATCGAA*G*G*G |
| 26 | AM1012-35 | G*G*G*G*CGATCGATCGAA*G*G*G*G |
| 27 | AM1012-23 | G*G*G*G*GCGATCGATCGAAG*G*G*G*G |
| 28 | AM1012-24 | G*G*G*G*G*GCGATCGATCGAAG*G*G*G*G*G |
| 29 | AM1012-25 | G*G*G*G*G*G*GCGATCGATCGAAG*G*G*G*G*G*G |
| 30 | AM1012-26 | G*G*G*G*G*G*G*GCGATCGATCGAAG*G*G*G*G*G*G*G |
| 31 | AM1012-27 | G*G*G*G*G*G*G*G*GCGATCGATCGAAG*G*G*G*G*G*G*G*G |
| 32 | AM1012-28 | G*G*G*G*G*G*G*G*G*GCGATCGATCGAAG*G*G*G*G*G*G*G*G*G |
| 33 | AM1012-29 | G*G*G*G*G*G*G*G*G*G*GCGATCGATCGAAG*G*G*G*G*G*G*G*G*G*G |
| 34 | AM1012-05-11 | G*G*GGCGATCGATCGAAAG*G*G*G*G*G |
| 35 | AM1012-05-12 | G*G*GGCGATCGATCGAAAAG*G*G*G*G*G |
| 36 | AM1012-05-13 | G*G*GGCGATCGATCGAAAAAG*G*G*G*G*G |
| 37 | AM1012-05-14 | G*G*G*G*GGCGATCGATCGAAG*G*G*G*G*G |
| 38 | AM1012-05-15 | G*G*G*GGCGATCGATCGAAG*G*G*G*G*G |
| 39 | AM1012-05-16 | G*G*GCGATCGATCGAAG*G*G*G*G*G |
| 40 | AM1012-05-17 | G*G*CGATCGATCGAAG*G*G*G*G*G |
| 41 | AM1012-05-18 | G*CGATCGATCGAAG*G*G*G*G*G |
| 42 | AM1012-05-19 | G*G*GGCGATCGATCGAAAAAAAAAAG*G*G*G*G*G |
| 43 | AM1012-05-20 | G*G*GGCGATCGATCGTTG*G*G*G*G*G |
| 44 | AM1012-05-21 | G*G*GGCGATCGATCGTTTTTG*G*G*G*G*G |
| 45 | AM1012-05-22 | G*G*GGCGATCGATCGTTTTTTTTTTG*G*G*G*G*G |
| 46 | AM1012-05-23 | G*G*GGCGATCGATCGCCG*G*G*G*G*G |
| 47 | AM1012-05-24 | G*G*GGCGATCGATCGCCCCCG*G*G*G*G*G |
| 48 | AM1012-05-25 | G*G*GGCGATCGATCGCCCCCCCCCCG*G*G*G*G*G |
| 49 | AM1012-05-26 | G*G*GGCGATCGATCGAG*G*G*G*G*G |
| 50 | AM1012-05-27 | G*G*GGCGATCGATCGG*G*G*G*G*G |
| 51 | 2216-01 | G*G*GGACGATCGTCG*G*G*G*G*G |
| 52 | 2216-02 | G*G*GACGATCGTCG*G*G*G*G*G |
| 53 | 2216-03 | G*GACGATCGTCG*G*G*G*G*G |
| 54 | 2216-04 | G*ACGATCGTCG*G*G*G*G*G |
| 55 | 2216-05 | G*G*GGGACGATCGTCAG*G*G*G*G*G |
| 56 | 2216-06 | G*G*GGGACGATCGTCAAG*G*G*G*G*G |
| 57 | 2216-07 | G*G*GGGACGATCGTCAAAG*G*G*G*G*G |
| 58 | 2216-08 | G*G*GGGACGATCGTCAAAAG*G*G*G*G*G |
| 59 | 2216-09 | G*G*GGGACGATCGTCAAAAAG*G*G*G*G*G |
| 60 | 2216-10 | G*G*GGGACGATCGTCAAAAAAAAAAG*G*G*G*G*G |
| 61 | 2216-11 | G*G*GGGACGATCGTCTTG*G*G*G*G*G |
| 62 | 2216-12 | G*G*GGGACGATCGTCTTTTTG*G*G*G*G*G |
| 63 | 2216-13 | G*G*GGGACGATCGTCTTTTTTTTTTG*G*G*G*G*G |
| 64 | 2216-14 | G*G*GGGACGATCGTCCCG*G*G*G*G*G |
| 65 | 2216-15 | G*G*GGGACGATCGTCCCCCCG*G*G*G*G*G |
| 66 | 2216-16 | G*G*GGGACGATCGTCCCCCCCCCCCG*G*G*G*G*G |
| 67 | D 19-01 | G*TGCATCGATGCAGG*G*G*G*G |
| 68 | D 19-02 | G*G*GTGCATCGATGCAGG*G*G*G*G |
| 69 | D19-03 | G*G*GGTGCATCGATGCAGG*G*G*G*G |
| 70 | D 19-04 | G*G*GGGTGCATCGATGCAGG*G*G*G*G |
| 71 | D 19-05 | G*G*TGCATCGATGCAAGG*G*G*G*G |
| 72 | D19-06 | G*G*TGCATCGATGCAAAGG*G*G*G*G |
| 73 | D 19-07 | G*G*TGCATCGATGCAAAAGG*G*G*G*G |
| 74 | D 19-08 | G*G*TGCATCGATGCAAAAAGG*G*G*G*G |
| 75 | D 19-09 | G*G*TGCATCGATGCAAAAAAGG*G*G*G*G |
| 76 | D19-10 | G*G*TGCATCGATGCAAAAAAAAAAAGG*G*G*G*G |
| 77 | D 19-11 | G*G*TGCATCGATGCATTGG*G*G*G*G |
| 78 | D19-12 | G*G*TGCATCGATGCATTTTTGG*G*G*G*G |
| 79 | D 19-13 | G*G*TGCATCGATGCATTTTTTTTTTGG*G*G*G*G |
| 80 | D 19-14 | G*G*TGCATCGATGCACCGG*G*G*G*G |
| 81 | D 19-15 | G*G*TGCATCGATGCACCCCCGG*G*G*G*G |
| 82 | D 19-16 | G*G*TGCATCGATGCACCCCCCCCCCGG*G*G*G*G |
| 83 | AM1012 | G*G*GGCGCGACGCGTCGCG*G*G*G*G*G |
| 84 | 2216 | G*G*GGGACGATCGTCG*G*G*G*G*G |
| *85* | D19 | G*G*TGCATCGATGCAGG*G*G*G*G |
| 86 | CpG 1018 | T*G*A*C*T*G*T*G*A*A*C*G*T*T*C*G*A*G*A*T*G*A |
| 87 | E41-1 | G*G*T*G*C*G*A*T*C*G*A*T*C*G*C*A*G*G*G*G*G*G |
| 88 | E41-2 | G*G*TGCGATCGATCGCAG*G*G*G*G*G |
| 89 | E41-3 | GGTGCGATCGATCGCAGGGGGG |

| | | |
|---|---|---|
| Note: * indicates thio modification. | | |

### Example 2: Multimerization and Activity Analysis of Different CpG ODNs

### (I) Multimerization Analysis of Different CpG ODNs:

Some of lyophilized powders of CpG ODN sequences obtained in Example 1 were analyzed for multimerization using high-performance liquid chromatography (HPLC).

### (II) Effect of different CpG ODNs on IFN-α secretion based on healthy human PBMCs:

(1) A certain amount of 100 µM CpG ODN solution was formulated as a stock solution of a certain concentration by using RPMI 1640 complete medium. Then, the CpG ODN sample solution was diluted 4 times with complete medium to prepare test samples of different concentrations.
(2) An appropriate amount of cryopreserved healthy human PBMCs were prepared.
(3) Cells were plated in a 96-well plate at 2 × 10⁵ cells/well, with samples of different concentrations added to each well. A PBS blank control well was also included.
(4) The plate was incubated at 37°C in a 5% CO₂ incubator for 16 h.
(5) The supernatant was collected by centrifugation.
(6) IFN-α was detected according to the IFN-α ELISA test instructions.

Analysis of the multimerization results (Tables 2-5) revealed that: (1) by modifying the number of Gs in sequences such as AM1012-05, CpG 2216, and CpG D19, the optimal multimerization state (lowest multimer content) for the corresponding sequences was obtained when the number of Gs at the 5' end was 3-4; (2) inserting 1-5 As between the core palindromic sequence and the poly-G at the 3' end of AM1012-05 could further reduce the multimer content; (3) inserting 1-10 Ts or Cs between the core palindromic sequence and the G at the 3' end of AM1012-05 could further reduce or otherwise increase the multimer content to a different extent; (4) inserting 1-10 As, Ts, or Cs between the core palindromic sequence and the G at the 3' end of CpG 2216 and CpG D19 could further reduce or otherwise increase the multimer content to a different extent.

Target cells for Type A CPG are pDCs, accounting for approximately 0.2-0.4% of PBMCs. The generated IFN-α mainly originates from pDCs. The activity evaluation results of this example are shown in Tables 2 to 5. Table 2 shows that, using "CGATCGATCG" as the core palindromic sequence of Type A CpG, adding different numbers of G before the C at its 5' end, thereby increasing its stimulatory activity on human PBMCs gradually as the number of G added increases, and reaching its strongest activity when 3 or 4 Gs are added, and then weakening with further increases in the number of Gs. Similarly, using the palindromic sequence "TGCATCGATGCA" of CpG D19 as the core sequence, adding different numbers of G before the T at its 5' end, thereby increasing its stimulatory activity on human PBMCs gradually as the number of G added increases, and reaching its strongest activity when 2-4 Gs are added, and then weakening with further increases in the number of Gs. When using the palindromic CpG 2216 sequence "GACGATCGTC" as the core sequence, adding different numbers of Gs before the G at the 5' end, thereby increasing its stimulatory activity on human PBMCs gradually as the number of G added increases, and reaching its strongest activity when 3 Gs are added, then weakening with further increases in the number of Gs.

Table 3 summarizes the multimerization and activity data of studies on the insertion of A between the 3' end of the palindromic structure of Type A CpG and the 5' end of poly-G. As can be seen, using "CGATCGATCG" as the core palindromic sequence of Type A CpG, gradually adding A after the sequence (before the 5' end of poly-G), thereby increasing its stimulatory activity on human PBMCs gradually as the number of A added increases, and reaching its strongest activity when 2-4 As are added, and then weakening with further increases in the number of As. Using "TGCATCGATGCA" of CpG D19 as the core palindromic sequence of Type A CpG, gradually adding A after the sequence (before the 5' end of poly-G), thereby increasing its stimulatory activity on human PBMCs as the number of A added increases, and reaching its strongest activity when 2 As are added, and then weakening with further increases in the number of As. Using "GACGATCGTC" of CpG 2216 as the core palindromic sequence of Type A CpG, gradually adding A after the sequence (before the 5' end of poly-G), thereby increasing its stimulatory activity on human PBMCs gradually as the number of A added increases, and reaching its strongest activity when 2 As are added, and then weakening with further increases in the number of As.

Table 4 summarizes the multimerization and activity data of studies on the insertion of T between the 3' end of the palindromic structure of Type A CpG and the 5' end of poly-G. When the A added to the 3' end of the palindromic sequence "CGATCGATCG" is replaced with T, adding 2 or 10 Ts to the end of the palindromic structure maintains the good activity of Type A CpG. When the A added to the 3' end of the palindromic sequence "TGCATCGATGCA" of CpG D19 is replaced with T, adding 2, 5, or 10 Ts to the end of the palindromic structure maintains the good activity of Type A CpG, especially D19-11, which has better activity than D19 when 2 Ts are added to it. When the A added to the 3' end of the palindromic sequence "GACGATCGTC" of CpG 2216 is replaced with T, adding 2, 5, or 10 Ts to the end of the palindromic structure maintains its good activity, especially CpG 2216-11, which has better activity than CpG 2216 when 2 Ts are added to CpG 2216.

Table 5 summarizes the multimerization and activity data of studies on the insertion of C between the 3' end of the palindromic structure of Type A CpG and the 5' end of poly-G. When the A added at the 3' end of the palindromic sequence "CGATCGATCG" is replaced with C, the activity of the sequence gradually decreases as the number of Cs at the end of the palindromic structure increases. When the A added at the 3' end of the CpG D19 palindromic sequence "TGCATCGATGCA" is replaced with C, the activity of the sequence gradually decreases as the number of Cs at the end of the palindromic structure increases. However, D19-14, which has two Cs added to D19, has better activity than D19. When the A added at the 3' end of the CpG2216 palindromic sequence "GACGATCGTC" is replaced with C, the activity of the sequence gradually decreases as the number of Cs at the end of the palindromic structure increases.

The present disclosure breaks through the conventional design concept of Type A CpGs, creatively establishing a spatial hinderance between poly-G and palindromes. This increases the energy for G-tetrad formation, making multimerization relatively difficult to form or relatively loose after formation. This successfully reduces the multimerization of Type A CpGs in solvents and enhances the activity of the designed novel Type A CpGs. Furthermore, exploration of the 5' end poly-G of conventional Type A CpGs revealed that when the number of Gs at 5' end is 3-4, the corresponding sequence exhibits the least multimerization and optimal activity. This pattern was verified in multiple CpG palindromic sequences. Based on these findings, AM1012-05 is the optimal newly designed sequence in this study. It has 4 Gs at the 5' end and two As inserted at the 3' end of the palindromic sequence, exhibiting both low multimerization and high activity, resulting in very high drugability.

This study breaks with conventional thinking by limiting the number of Gs (to 3-4) at the 5' end of the palindromic sequence of Type A CpG and inserting an appropriate number of As and Ts (preferably 2) between the palindromic sequence and the poly-G at the 3' end, thereby altering the multimerization of Type A CpG and improving its activity.

**Table 2. Relationship between the number of Gs at 5'end of Type A CpG palindromic sequences and multimerization and activity.**

| ID | Number of Gs | Multimerization rate (%) | Activity (IFN-α, pg/mL) |
|---|---|---|---|
| AM1012-05-18 | 1 | 16.01 | 287.8±149.5^{a} |
| AM1012-05-17 | 2 | 7.12 | 188.6±69.5^{a} |
| AM1012-05-16 | 3 | 3.03 | 615.3±58.3^{a} |
| AM1012-05 | 4 | 0.7 | 573.6±47.2^{a} |
| AM1012-05-15 | 5 | 10.63 | 337.8±109.7^{a} |
| AM1012-05-14 | 6 | 26.38 | 107.8±75.8^{a} |
| D19-01 | 1 | 17.11 | 276.3±96.6^{b} |
| D19 | 2 | 4.94 | 531.1±137.6^{b} |
| D 19-02 | 3 | 0.61 | 481.1±48.1^{b} |
| D 19-03 | 4 | 0.68 | 527.1±106.7^{b} |
| D19-04 | 5 | 6.05 | 203.4±45.6^{b} |
| 2216-04 | 1 | 23.28 | 256.8±155.4^{b} |
| 2216-03 | 2 | 16.52 | 349.1±122.4^{b} |
| 2216-02 | 3 | 2.17 | 323.5±145.2^{b} |
| 2216-01 | 4 | 0.83 | 548.5±40.4^{b} |
| 2216 | 5 | 4.21 | 310.2±74.1^{b} |

| | | | |
|---|---|---|---|
| Note: a, 0.2µM; b, 8µM | | | |

**Table 3. Relationship between the number of As at the 3' end (before poly-G) of Type A CpG palindromic sequences and multimerization and activity.**

| ID | Number of As | Multimerization rate (%) | Activity (IFN-α, pg/mL) |
|---|---|---|---|
| AM1012-05-27 | 0 | 1.9 | 506.5±172.4^{b} |
| AM1012-05-26 | 1 | 0.9 | 616.0±35.1^{b} |
| AM1012-05 | 2 | 0.7 | 630.8±72.3 ^{b} |
| AM1012-05-11 | 3 | 0.37 | 781.2±68.2^{b} |
| AM1012-05-12 | 4 | 0.25 | 656.5±132.2^{b} |
| AM1012-05-13 | 5 | 0.93 | 578.9±133.1^{b} |
| AM1012-05-19 | 10 | 13.70 | 537.0±309.7^{b} |
| D19 | 0 | 4.94 | 357.8±48.0^{b} |
| D19-05 | 1 | 6.96 | 425.1±101.2^{b} |
| D19-06 | 2 | 7.75 | 456.6±101.8^{b} |
| D19-07 | 3 | 15.33 | 368.2±82.0^{b} |
| D19-08 | 4 | 19.22 | 205.8±184.6^{b} |
| D19-09 | 5 | 22.03 | 222.8±304.1 |
| D19-10 | 10 | 13.32 | 38.7±9.6^{b} |
| 2216 | 0 | 4.21 | 249.5±104.0^{b} |
| 2216-05 | 1 | 3.97 | 654.1±24.0^{b} |
| 2216-06 | 2 | 7.53 | 532.5±57.6^{b} |
| 2216-07 | 3 | 8.52 | 514.8±60.6^{b} |
| 2216-08 | 4 | 12.13 | 468.1±122.0^{b} |
| 2216-09 | 5 | 17.03 | 294.1±38.8^{b} |
| 2216-10 | 10 | 51.23 | 333.1±107.0^{b} |

| | | | |
|---|---|---|---|
| Note: b, 0.8µM | | | |

**Table 4. Relationship between the number of Ts at the 3' end (before poly-G) of Type A CpG palindromic sequences and multimerization and activity.**

| ID | Number of Ts | Multimerization rate (%) | Activity (IFN-α, pg/mL) |
|---|---|---|---|
| AM1012-05 | 0 | 0.7 | 478.4±44.9^{b} |
| AM1012-05-20 | 2 | 1.7 | 531.7±11.9^{b} |
| AM1012-05-21 | 5 | 1.26 | 458.1±17.3^{b} |
| AM1012-05-22 | 10 | 10.38 | 576.6±115.7^{b} |
| D19 | 0 | 4.94 | 345.9±93.1^{b} |
| D19-11 | 2 | 15.22 | 502.3±81.4^{b} |
| D19-12 | 5 | 20.75 | 398.4±38.8^{b} |
| D19-13 | 10 | 23.56 | 228.1±42.6^{b} |
| 2216 | 0 | 4.21 | 435.8±129.6^{b} |
| 2216-11 | 2 | 11.41 | 525.5±64.0^{b} |
| 2216-12 | 5 | 19.68 | 455.8±24.3^{b} |
| 2216-13 | 10 | 44.83 | 405.8±192.3^{b} |

| | | | |
|---|---|---|---|
| Note: b, 0.8µM | | | |

**Table 5. Relationship between the number of Cs at the 3' end (before poly-G) of Type A CpG palindromic sequences and multimerization and activity.**

| ID | Number of Cs | Multimerization rate (%) | Activity (IFN-α, pg/mL) |
|---|---|---|---|
| AM1012-05 | 0 | 0.7 | 478.4±44.9^{b} |
| AM1012-05-23 | 2 | 0.84 | 399.3±108.4^{b} |
| AM1012-05-24 | 5 | 5.75 | 258.4±49.6^{b} |
| AM1012-05-25 | 10 | 0 | 10.5±1.4^{b} |
| D19 | 0 | 4.94 | 345.9±93.1^{b} |
| D19-14 | 2 | 6.42 | 552.0±134.2^{b} |
| D19-15 | 5 | 3.74 | 49.0±2.4 ^{b} |
| D19-16 | 10 | 0 | 45.4±1.0^{b} |
| 2216 | 0 | 4.21 | 435.8±129.6^{b} |
| 2216-14 | 2 | 2.54 | 150.5±39.3^{b} |
| 2216-15 | 5 | 6.5 | 118.8±44.7^{b} |
| 2216-16 | 10 | 0 | 15.2±0.5 ^{b} |

| | | | |
|---|---|---|---|
| Note: b, 0.8µM | | | |

### Example 3: Cytokine Release in NK-92 Cells Stimulated with Different CpG ODNs

(1) NK-92 cells are human NK cell lines expressing the TLR-9 receptor, commonly used to assess the function of TLR-9 agonists. In this experiment, NK-92 cells were cultured and amplified to the required cell count according to the cell instructions, and then resuspended in a certain amount of complete culture medium.
(2) 20 µL of cell suspension was mixed with trypan blue at a 1:1 ratio and counted using a cell counter to determine cell viability and density.
(3) 2.5 × 10⁵ cells/well were plated in a 96-well plate, with different concentrations of CpG samples (sequences in Table 1) added to each well, with 3 replicates per group.
(4) The cells were incubated at 37°C in a 5% CO₂ incubator for 72 hours.
(5) After cell culture, the cells were centrifuged at 1200 rpm for 5 min, and 100 µL of cell-free supernatant was collected; the secretion of IFN-γ in the supernatant was detected according to the ELISA kit instructions.

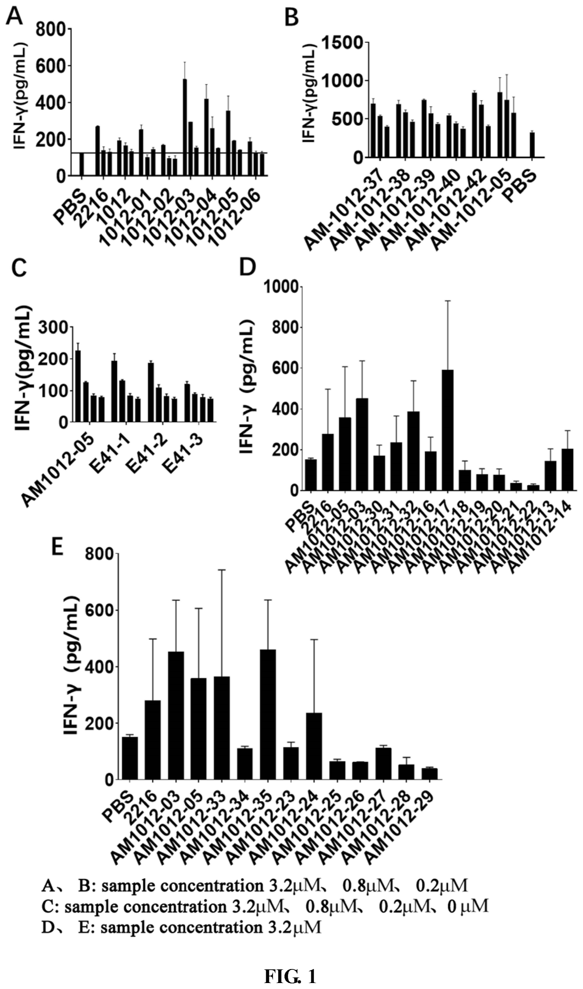

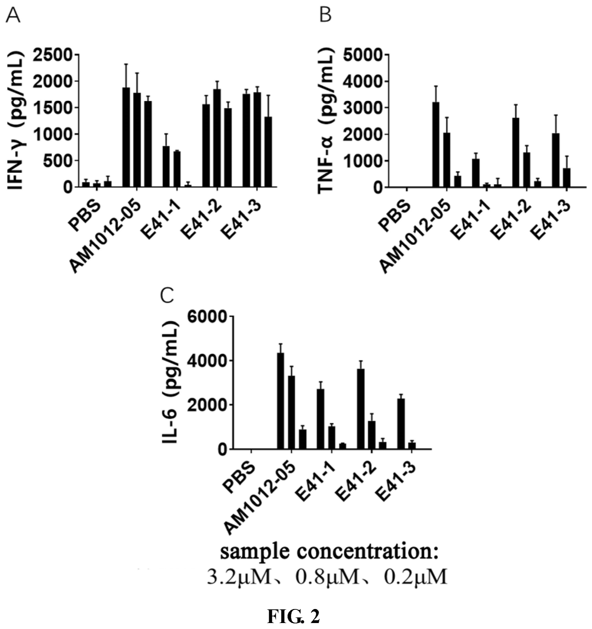

The experimental results are shown in Figure 1. All the designed CpG sequences in Table 1 of the present disclosure can effectively stimulate the secretion of IFN-γ of NK cells. Among them, the stimulatory effects of AM1012-03, AM1012-04, AM1012-05, AM1012-17, and AM1012-35 sequences are particularly significant. In addition, the stimulatory effect of AM1012-05 is better than that of E41-1 and E41-2, and significantly better than that of E41-3. Furthermore, the experimental results in Figure 2 (Panels D and E) show that increasing or decreasing the number of Gs on both sides of the core sequence of the present disclosure changes the immunostimulatory activity of the sequence. When the number of Gs on both sides of the core sequence reaches a certain amount, the sequence can provide the best immune activation effect.

### Example 4: Effects of CpG Sequences on the Secretion of IFN-γ, IL-6, and TNF-α Based on Spleen Cells from SD Rats

(1) A certain amount of 100 µM AM1012-05, E41-1, E41-2, and E41-3 solutions were formulated as a stock solution of a certain concentration using RPMI 1640 complete medium. Then, the solution was diluted with complete medium in a 4-fold ratio to prepare test samples of different concentrations.
(2) An appropriate amount of fresh SD rat spleen cells were obtained.
(3) 1 × 10⁶ cells/well were plated in a 96-well plate, with different concentrations (final concentrations of 3.2µM, 0.8µM, and 0.2µM, respectively) of AM1012-05 samples added to each well, with 3 replicates per group. PBS blank control wells were also included.
(4) The cells were incubated at 37°C in a 5% CO₂ incubator for 16 or 72 hours.
(5) The supernatant was collected by centrifugation.
(6) IFN-γ, IL-6 and IFN-α were detected according to the ELISA test instructions for different indicators.

The experimental results are shown in Figure 2. The designed AM1012-05 and E41-1, E41-2 and E41-3 can significantly stimulate the secretion of IFN-γ, TNF-α and IL-6 in spleen cells of SD rats, and have good dose-dependent effects. However, AM1012-05 showed significantly better immune activation ability in spleen cells of SD rats than E41-1, E41-2, and E41-3. Combined with Panel C in Figure 2, it can be found that AM1012-05 has a better immune activation ability.

### Example 5: Effects of AM1012-05 Sequence on NK-92 Cytokine Release

(1) NK-92 cells were prepared to the required cell volume as described in Example 3, and resuspended in a certain amount of complete culture medium.
(2) 20 µL of cell suspension was mixed with trypan blue at a 1:1 ratio and counted using a cell counter to determine cell viability and density.
(3) AM1012-05 sample was formulated into a 100 µM solution with PBS.
(4) A certain amount of 100 µM AM1012-05 solution was formulated as a stock solution of a certain concentration using complete medium. Then, the AM1012-05 solution was diluted with complete medium in a 2-fold ratio to prepare test AM1012-05 samples of different concentrations.
(5) 2.5 × 10⁵ cells/well were plated in a 96-well plate, with different concentrations (final concentrations of 20µM, 10µM, 5µM, 2.5µM, 1.25µM, 0.63µM, 0.31µM, 0.16µM, and 0.08µM, respectively) of AM1012-05 samples added to each well, with 3 replicates per group. PBS blank control wells were also included.
(6) The cells were incubated at 37°C in a 5% CO₂ incubator for 72 hours.
(7) After cell culture, the cells were centrifuged at 1200 rpm for 5 min, and 100 µL of cell-free supernatant was collected; the secretion of IFN-γ in the supernatant was detected according to the ELISA kit instructions.

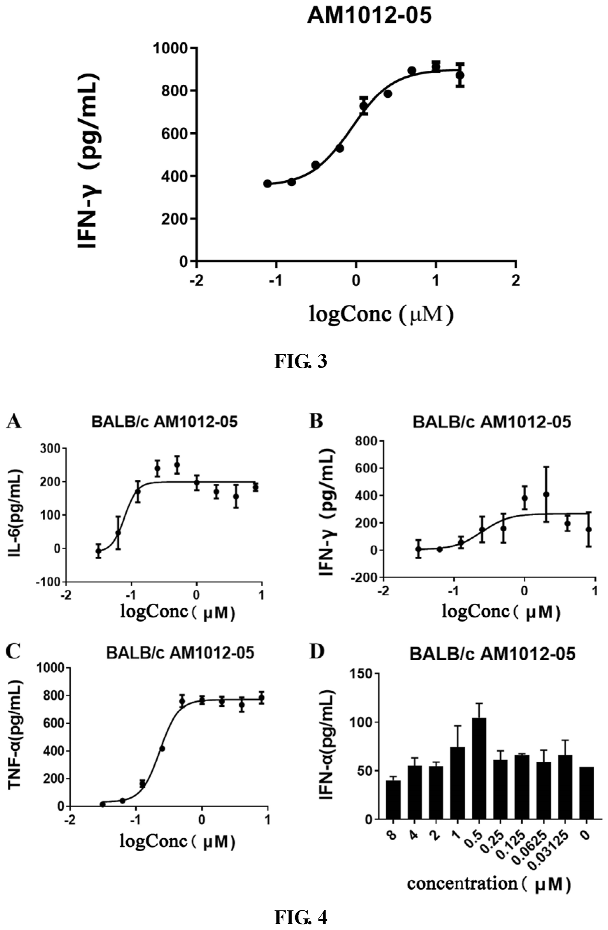

The experimental results are shown in Figure 3. The AM1012-05 sequence can effectively stimulate the secretion of IFN-γ in NK-92 cells, with good dose-dependent effect. Since NK-92 cells express TLR9, this experiment also verified that the immune-activating activity of AM1012-05 is achieved by binding with TLR9 each other.

### Example 6: Effects of AM1012-05 on the Secretion of IL-6, IFN-γ, TNF-α, etc., Based on BALB/c Mouse Spleen Cells.

(1) A certain amount of 100 µM AM1012-05 solution was formulated as a stock solution of a certain concentration using RPMI 1640 complete medium. Then, the AM1012-05 solution was diluted with complete medium in a 2-fold ratio to prepare test AM1012-05 samples of different concentrations.
(2) An appropriate amount of fresh BALB/c mouse spleen cells were obtained.
(3) 1 × 10⁶ cells/well were plated in a 96-well plate, with different concentrations (final concentrations of 8µM, 4µM, 2µM, 1µM, 0.5µM, 0.25µM, 0.13µM, 0.06µM, and 0.03µM, respectively) of AM1012-05 samples added to each well, with 3 replicates per group. PBS blank control wells were also included.
(4) The cells were incubated at 37°C in a 5% CO₂ incubator for 16 or 72 hours.
(5) The supernatant was collected by centrifugation.
(6) IL-6, IFN-γ, TNF-α and IFN-α were detected according to the ELISA test instructions for different indicators.

The experimental results are shown in Figure 4. The designed AM1012-05 sequence can effectively stimulate the secretion of cytokine in BALB/c mouse spleen cells, with a certain dose-dependency.

### Example 7: Effects of AM1012-05 on the Secretion of IL-6, IFN-γ, TNF-α, IFN-α, etc., Based on C57BL6 Mouse Spleen Cells.

(1) A certain amount of 100 µM AM1012-05 solution was formulated as a stock solution of a certain concentration using RPMI 1640 complete medium. Then, the AM1012-05 solution was diluted with complete medium in a 2-fold ratio to prepare test AM1012-05 samples of different concentrations.
(2) An appropriate amount of fresh C57BL6 mouse spleen cells were obtained.
(3) 1 × 10⁶ cells/well were plated in a 96-well plate, with different concentrations (final concentrations of 8µM, 4µM, 2µM, 1µM, 0.5µM, 0.25µM, 0.13µM, 0.06µM, and 0.03µM, respectively) of AM1012-05 samples added to each well, with 3 replicates per group. PBS blank control wells were also included.
(4) The cells were incubated at 37°C in a 5% CO₂ incubator for 16 or 72 hours.
(5) The supernatant was collected by centrifugation.
(6) IL-6, INF-γ, TNF-α and IFN-α were detected according to the ELISA test instructions for different indicators.

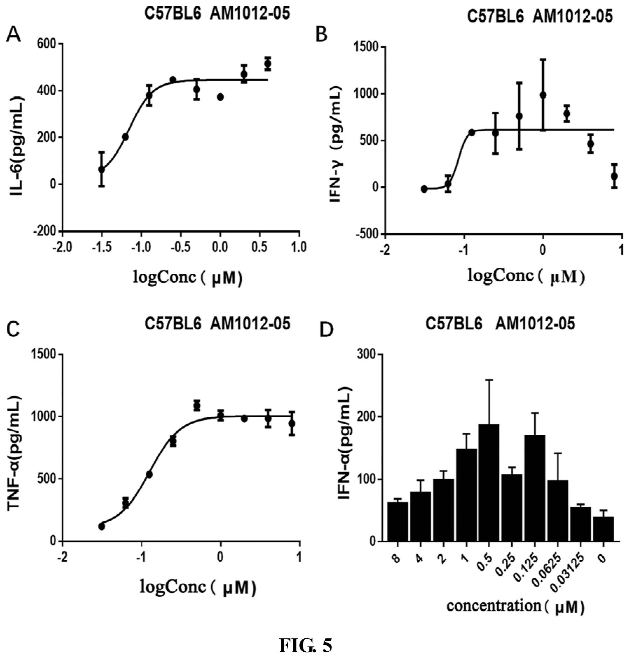

The experimental results are shown in Figure 5. The designed AM1012-05 sequence can effectively stimulate the secretion of cytokine in C57BL6 mouse spleen cells, with a certain dose-dependency.

### Example 8: Effects of AM1012-05 on IFN-α, IFN-γ, TNF-α, and CXCL-10 mRNA Transcription in Cotton rat Spleen Cells.

(1) A certain amount of 100 µM AM1012-05 solution was formulated as a stock solution of a certain concentration using RPMI 1640 complete medium. Then, the AM1012-05 solution was diluted with complete medium in a 2-fold ratio to prepare test AM1012-05 samples of different concentrations.
(2) An appropriate amount of cryopreserved cotton rat spleen cells were obtained.
(3) 5 × 10⁶ cells/well were plated in a 96-well plate, with different concentrations (final concentrations of 0.2µM, 0.8µM, and 3.2µM, respectively) of AM1012-05 samples added to each well, with 3 replicates per group. PBS blank control wells were also included.
(4) The plate was incubated at 37°C in a 5% CO₂ incubator for 3 h.
(5) Cells were collected and total RNAs were extracted from samples according to the RNA extraction kit instructions. The total RNA concentration was determined using Nanodrop.
(6) The RNAs were reverse transcribed into cDNAs according to the reverse transcription kit instructions.
(7) The expression levels of relevant cytokines in each sample were analyzed according to the RT-qPCR kit instructions.

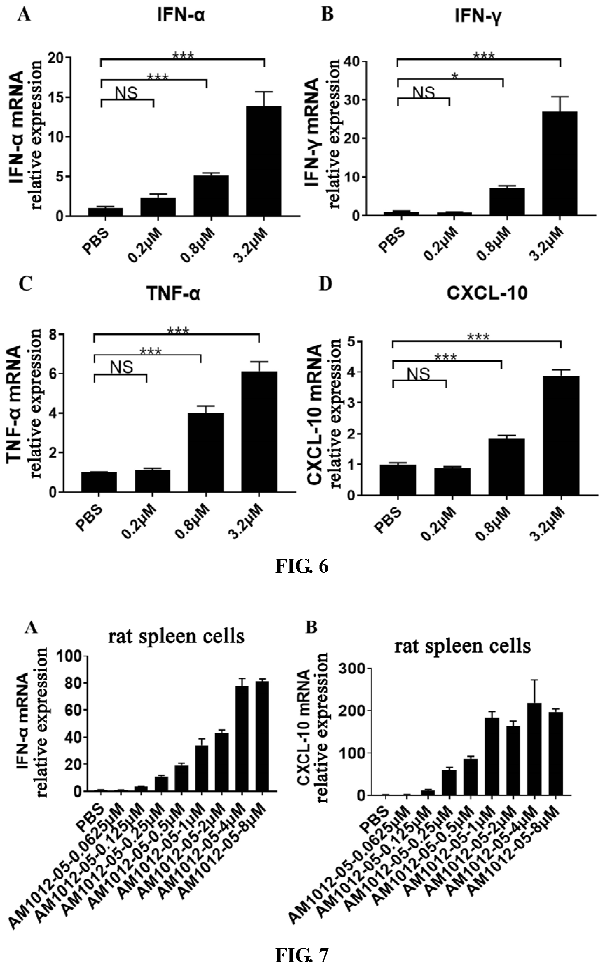

The experimental results are shown in Figure 6. The designed AM1012-05 sequence can effectively stimulate the secretion of immune-related cytokine in cotton rat spleen cells, with a good dose-dependency. Cotton rat is a commonly used animal model for respiratory syncytial virus (RSV) infection, which lays the foundation for evaluating the anti-RSV effect of AM1012-05 using the same.

### Example 9: Effects of AM1012-05 on IFN-α and CXCL-10 mRNA Transcription in SD Rat Spleen Cells.

(1) A certain amount of 100 µM AM1012-05 solution was formulated as a stock solution of a certain concentration using RPMI 1640 complete medium. Then, the AM1012-05 solution was diluted with complete medium in a 2-fold ratio to prepare test AM1012-05 samples of different concentrations.
(2) An appropriate amount of fresh rat spleen cells were obtained.
(3) Cells were plated in a 96-well plate at 5 × 10⁶ cells/well, with AM1012-05 samples having different concentrations added to each well, with 3 replicates per group. A PBS blank control well was also included.
(4) The plate was incubated at 37°C in a 5% CO₂ incubator for 3 h.
(5) Cells were collected and total RNAs were extracted from samples according to the RNA extraction kit instructions. The total RNA concentration was determined using Nanodrop.
(6) The RNAs were reverse transcribed into cDNAs according to the reverse transcription kit instructions.
(7) The expression levels of relevant cytokines in each sample were analyzed according to the RT-qPCR kit instructions.

The experimental results are shown in Figure 7. The designed AM1012-05 sequence can effectively stimulate the transcription of IFN-λ and CXCL-10 mRNAs in SD rat spleen cells, with a good dose-dependency.

### Example 10: Effects of AM1012-05 on the Secretion of IFN-γ, IL-6, and TNF-α based on Spleen Cells from SD Rats

(1) A certain amount of 100 µM AM1012-05 solution was formulated as a stock solution of a certain concentration using RPMI 1640 complete medium. Then, the AM1012-05 solution was diluted with complete medium in a 2-fold ratio to prepare test AM1012-05 samples of different concentrations.
(2) An appropriate amount of fresh SD rat spleen cells were obtained.
(3) 1 × 10⁶ cells/well were plated in a 96-well plate, with different concentrations (final concentrations of 20µM, 10µM, 5µM, 2.5µM, 1.25µM, 0.625µM, 0.3125µM, 0.1562µM, and 0.087µM, respectively) of AM1012-05 samples added to each well, with 3 replicates per group. PBS blank control wells were also included.
(4) The cells were incubated at 37°C in a 5% CO₂ incubator for 16 or 72 hours.
(5) The supernatant was collected by centrifugation.
(6) IFN-γ, IL-6 and IFN-α were detected according to the ELISA test instructions for different indicators.

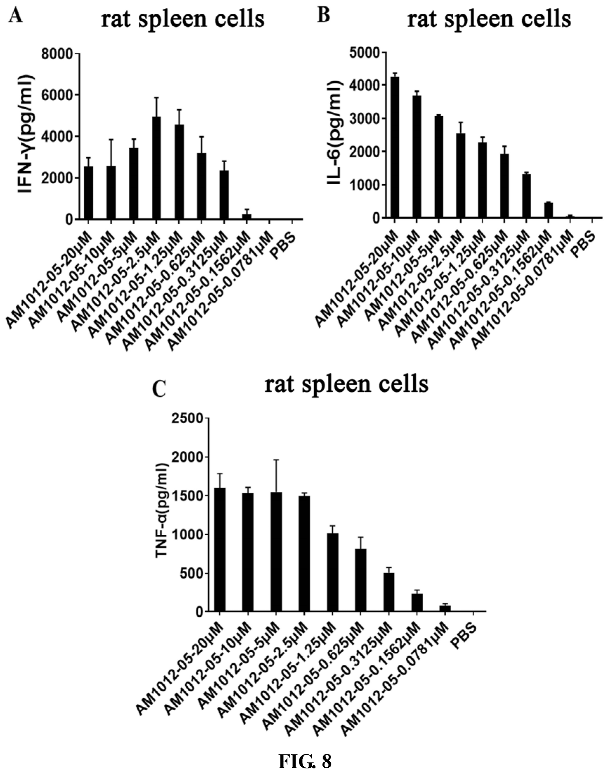

The experimental results are shown in Figure 8. The designed AM1012-05 sequence can effectively stimulate the secretion of IFN-γ, IL-6 and TNF-α in SD rat spleen cells, with a good dose-dependency. SD rat is a commonly used animal model for preclinical drug toxicology evaluation. Combined with the data from Example 9, the results of this experiment lay the foundation for conducting a safety evaluation experiment of AM1012-05 using SD rats.

### Example 11: Effects of AM1012-05 on Secretion of IFN-α, IFN-y, IFN-λ, IL-6, TNF-α, and CXCL-10 in Beagle Dog PBMCs.

(1) A certain amount of 100 µM AM1012-05 solution was formulated as a stock solution of a certain concentration using RPMI 1640 complete medium. Then, the AM1012-05 solution was diluted with complete medium in a 4-fold ratio to prepare test AM1012-05 samples of different concentrations.
(2) An appropriate amount of fresh beagle dog PBMCs were obtained.
(3) 5 × 10⁶ cells/well were plated in a 96-well plate, with different concentrations (final concentrations of 0.2µM, 0.8µM, and 3.2µM, respectively) of AM1012-05 samples added to each well, with 3 replicates per group. PBS blank control wells were also included.
(4) The plate was incubated at 37°C in a 5% CO₂ incubator for 6 h.
(5) Cells were collected and total RNAs were extracted from samples according to the RNA extraction kit instructions. The total RNA concentration was determined using Nanodrop.
(6) The RNAs were reverse transcribed into cDNAs according to the reverse transcription kit instructions.
(7) The expression levels of relevant cytokines in each sample were analyzed according to the RT-qPCR kit instructions.

The experimental results are shown in Figure 9. The designed AM1012-05 sequence can effectively stimulate the secretion of immune-related cytokine in beagle dog PBMCs, with a good dose-dependency. Beagle dog is a commonly used animal model for preclinical drug toxicology evaluation, which lays the foundation for conducting a safety evaluation experiment of AM1012-05 using beagle dogs. Furthermore, the efficacy demonstrated by AM1012-05 on the Beagle PBMC suggests its significant potential as an adjuvant for pet vaccines or in the anti-infection application.

### Example 12: Effects of AM1012-05 and Related Sequences on Secretion of IFN-α, IFN-γ, IFN-λ, TNF-α, IL-6, and CXCL-10 in Healthy Human PBMCs.

(1) A certain amount of 100 µM solutions of AM1012-05 and related sequences were formulated as a stock solution of a certain concentration using RPMI 1640 complete medium. Then, the solutions of AM1012-05 and related sequences were diluted with complete medium in a 4-fold ratio to prepare test samples of different concentrations.

An appropriate amount of cryopreserved healthy human PBMCs were prepared.

(3) Cells were plated in a 96-well plate at 2 × 10⁵ cells/well, with samples of different concentrations added to each well, with 3 replicates per group. A PBS blank control well was also included.

(4) The plate was incubated at 37°C in a 5% CO₂ incubator for 16 h.

(5) The supernatant was collected by centrifugation.

(6) Cytokine was detected according to the ELISA test instructions.

The experimental results are shown in Figure 10. AM1012-05 can stimulate the secretion of IFN-λ (Figure 10, Panel A), IFN-γ (Figure 10, Panel B), IFN-λ (Figure 10, Panel C), TNF-α (Figure 10, Panel D), IL-6 (Figure 10, Panel E), and CXCL-10 (Figure 10, Panel F) from PBMCs in a dose-dependent manner. Furthermore, AM1012-05 and related sequences showed significantly better activation of IFN-λ than CpG 2216 and D19 (Figure 10, Panel G). Among them, AM1012-05 showed a significant effect in promoting IFN-α secretion, with an EC50 of 0.19 µM and an optimal stimulation concentration of 0.5 µM. 1 µM AM1012-05 also showed a significant effect in promoting IFN-γ secretion, and 0.25 µM AM1012-05 showed a significant effect in promoting IFN-λ secretion. This demonstrates that AM1012-05 has a very strong antiviral potential. AM1012-05 can stimulate TNF-α secretion in healthy human PBMCs with a good dose dependence, but its ability to stimulate TNF-α is significantly lower than its ability to stimulate IFN-α (EC50 is 0.19 and 2.46 µM, respectively). This indicates that when AM1012-05 exerts highly effective antiviral activity (IFN-α, IFN-γ, and IFN-λ) at lower doses, it does not trigger inflammatory responses as represented by TNF-α and IL-6.

### Example 13: Serum Stability Detection of CpG ODNs

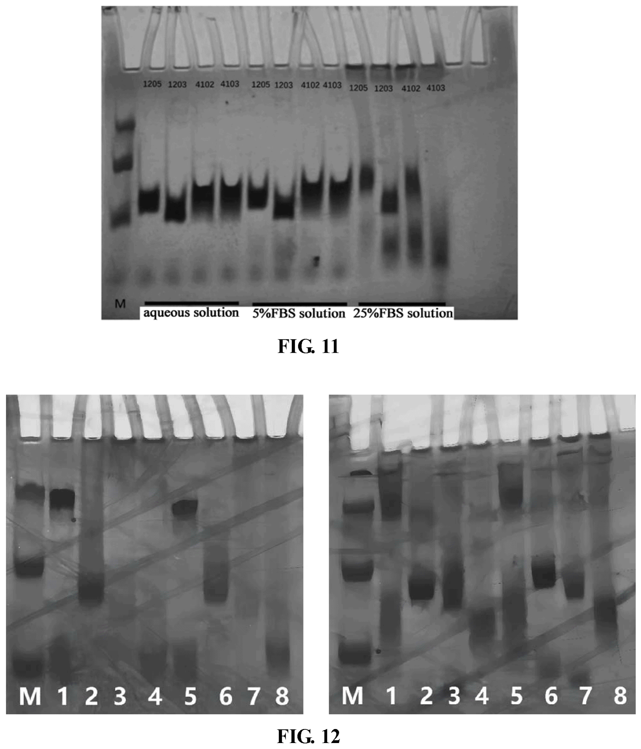

This experiment investigated the degree of sequence damage caused by fetal bovine serum (FBS) using polyacrylamide gel electrophoresis (PAGE). The specific procedure was as follows: AM1012-05, AM1012-03, E41-2, and E41-3 were each formulated as 25 OD/mL aqueous solutions. 10 µL of 10% FBS was added, and the solutions were incubated at 37°C for a certain period. After incubation, the degree of sequence damage was detected using PAGE. The results, as shown in Figure 11, indicate that AM1012-05 (1205) remained stable in the 25% FBS solution. E41-3 showed the greatest degradation (4103), AM1012-03 (1203) and E41-2 (4102) showed moderate degradation, and AM1012-05 (1205) showed the least degradation. Therefore, compared to E41-2 and E41-3, the AM1012-05 sample has better stability.

### Example 14: Multimerization Distribution Analysis of Different CpG ODN Multimers

3 mg of AM1012-03, E41-2, E41-3, and AM1012-05 were added to 1 ml of deionized water to obtain a clear solution.

The above four solutions were divided into two groups: one group was placed at room temperature, and the other was heated in a 95°C water bath for 30 minutes and then rapidly cooled to below 4°C with ice water. Equal volumes of 800 mM sodium chloride solution were added to each of the above eight solutions to provide a 400 mM sodium ion environment. After incubation for 24 hours, precipitates were separated by centrifugation. The precipitates were redissolved in water to prepare a clear solution. The multimerization distribution of each sample was tested using polyacrylamide gel electrophoresis (PAGE).

The electrophoresis results are shown in Figure 12. In the figure, M represents 20, 40, and 80 bases. Lanes 1, 2, 3, and 4 correspond to samples AM1012-03, E41-2, E41-3, and AM1012-05 placed at room temperature, respectively. Lanes 5, 6, 7, and 8 correspond to samples AM1012-03, E41-2, E41-3, and AM1012-05 that were heated in a 95°C water bath for 30 minutes and then rapidly cooled to below 4°C with ice water, respectively. The left panel shows the electrophoresis results after the precipitate has dissolved in water for 2 hours, and the right panel shows the electrophoresis results after the same sample solution has been left for 24 hours.

Literature reports indicate that the poly-G sequence at the 5' or 3' end of Type A CpG can lead to the formation of multimers of Type A CpG with the assistance of metal ions, which can accumulate and cause precipitation. The controllability of the multimerization is a key indicator for evaluating the drugability of Type A CpG (Kerkmann et al., J Biol Chem. 2005 Mar 4;280(9):8086-93).

In this experiment, all samples incubated in a 400 mM sodium ion environment formed precipitates. After the precipitates were placed back into water, the high-polymerized state of Type A CpG gradually depolymerized in the absence of metal ions and could be dissolved again. The low-polymerized forms such as dimers and tetramers also have a certain degree of solubility in water. Therefore, observing the redissolving samples at different time points can maximize the observation of the (lower) polymerized state distribution of each Type A CpG and its trend over time.

For samples at room temperature, the redissolved solution of AM1012-03 was mainly composed of tetramers and monomers after 2 hours, and showed diffuse bands after 24 hours; the redissolved solution of E-41-2 was mainly composed of dimers after 2 hours, and was still mainly composed of dimers after 24 hours, but tetramers gradually appeared; the redissolved solution of E-41-3 showed dimers and polymers (larger than tetramers) after 2 hours, and showed diffuse bands mainly composed of dimers after 24 hours; the redissolved solution of AM1012-05 was mostly in the monomer state after 2 hours and 24 hours, with only a small number of multimers.

For samples which were heated in a 95°C water bath for 30 minutes and then rapidly cooled to below 4°C with ice water, the redissolved solution of AM1012-03 was mainly composed of tetramers and monomers after 2 hours, and diffuse bands after 24 hours. The redissolved solution of E-41-2 was mainly composed of dimers after 2 hours, and remained predominantly dimers after 24 hours, but tetramers and monomers gradually appeared. The redissolved solution of E-41-3 was a collection of different multimers after 2 hours, and after 24 hours, it was diffuse bands predominantly of monomers and dimers. In the redissolved solution of AM1012-05, most samples remained in a monomeric state after 2 hours and 24 hours, with only a small number of multimers.

Based on the above results, AM1012-05, compared to other Type A CpG monomers, is more likely to depolymerize back to its monomeric state under the same condition. Other Type A CpGs can transform between monomer, dimer, tetramer, or other polymeric forms. Therefore, in practical formulation applications, as long as the concentration of the metal salt is properly controlled, AM1012-05 can stably be maintained in its monomeric state. After the formulation enters the human body, at physiologically appropriate salt concentrations, AM1012-05 can generate the polymeric state characteristic specific for Type A CpGs, thus producing its therapeutic effect.

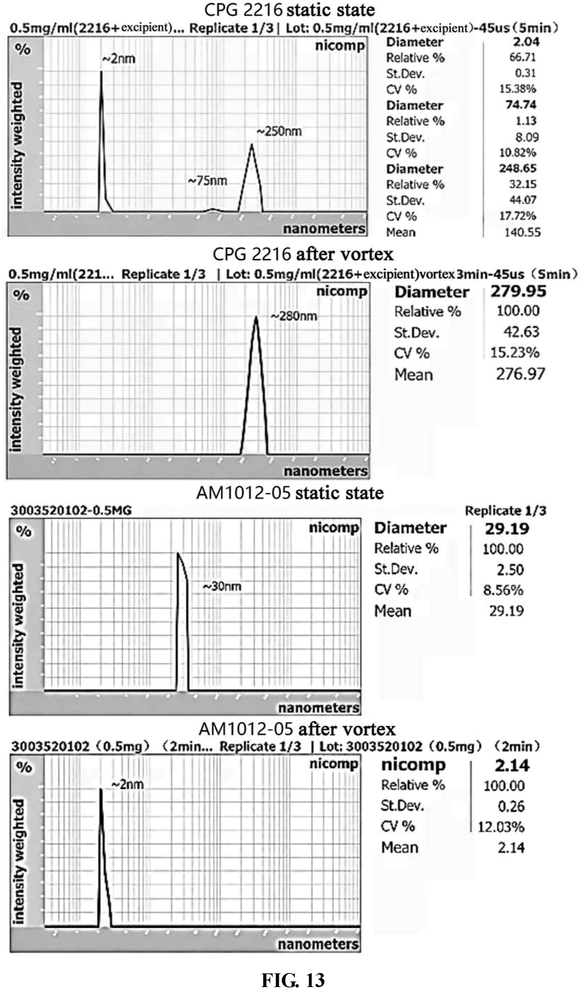

Meanwhile, CpG 2216 and AM1012-05 were formulated as 0.5 mg/mL solutions using 10 mM phosphate buffer solution. After standing overnight, the particle size of CpG was measured using a Nicomp Z3000 laser particle size analyzer. After measurement, the solutions were vortexed for 3 minutes, and the particle size was measured again. The results, shown in Figure 13, indicate that the particle size distribution of CpG 2216 before vortexing was approximately 2 nm, 75 nm, and 250 nm, while after vortexing, the particle size of CpG 2216 was predominantly around 280 nm. In contrast, the particle size of AM1012-05 was approximately 30 nm in the static state, while after vortexing, the particle size of AM1012-05 was predominantly around 2 nm.

Laser particle size analyzer results showed the multimerization state of CpG under natural conditions. In a 10 mM phosphate buffer solution, AM1012-05 exhibited a loose multimerized state under static conditions; however, the multimers were broken up into monomers under mechanical force. In contrast, CpG 2216, in the same 10 mM phosphate buffer solution, exhibited a dispersed complex polymer that could not be broken up under mechanical force.

Figure 13 shows that CpG 2216, being a conventional Type A CpG, lacks the additional deoxyribonucleotides between the 3' end of its palindromic structure and the 5' end of its poly-G, resulting in a lack of spatial hinderance. This reduces the energy required for G-tetrad formation, leading to tightly bound multimers that cannot be easily broken up by mechanical forces. In contrast, AM1012-05 incorporates two As between the 3' end of its palindromic structure and the 5' end of its poly-G, creating spatial hinderance and increasing the energy required for G-tetrad formation. This makes the multimers relatively difficult to form or relatively loose once formed. Therefore, under natural static conditions, AM1012-05 exhibits a uniformly distributed multimer equilibrium state of approximately 30 nm, significantly enhancing its drugability compared to conventional Type A CpGs.

### Example 15: Simulation Analysis and Comparison of Secondary Structures of Different Sequences

The secondary structures of CpG 2216, E41-1, AM1012-3, and AM1012-5 sequences were predicted using an online modeling tool (https://ma.urmc.rochester.edu/). The secondary structures of each sequence under different temperature conditions were obtained by adjusting the temperature parameters.

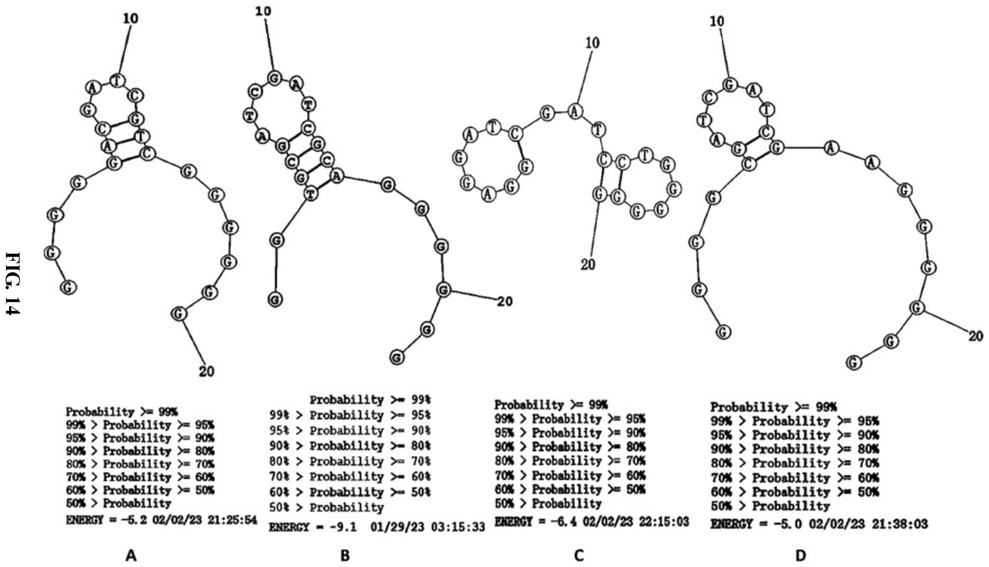

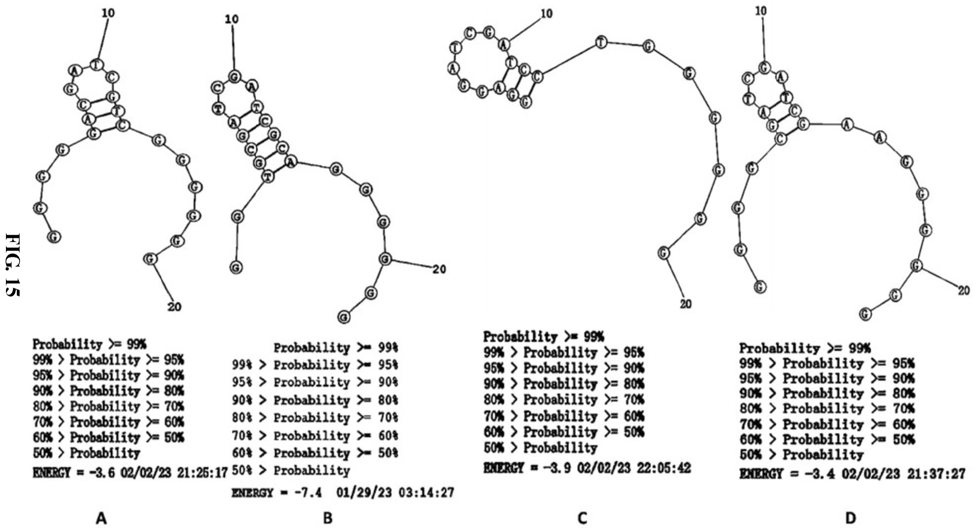

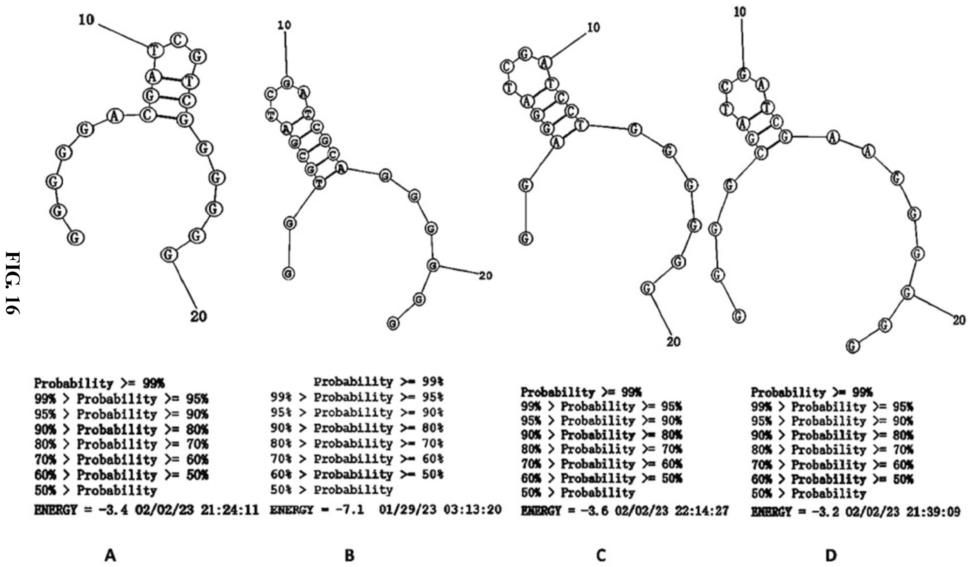

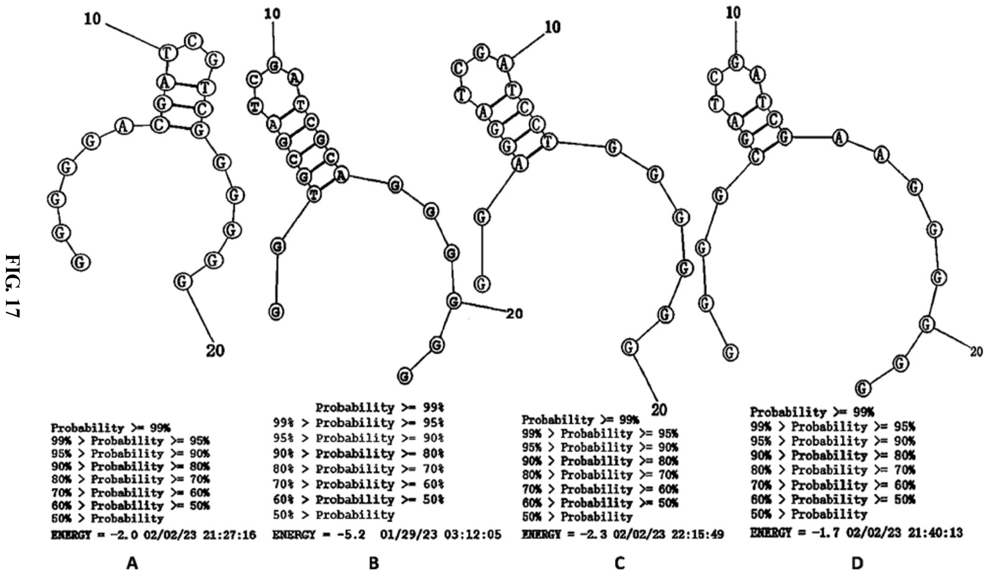

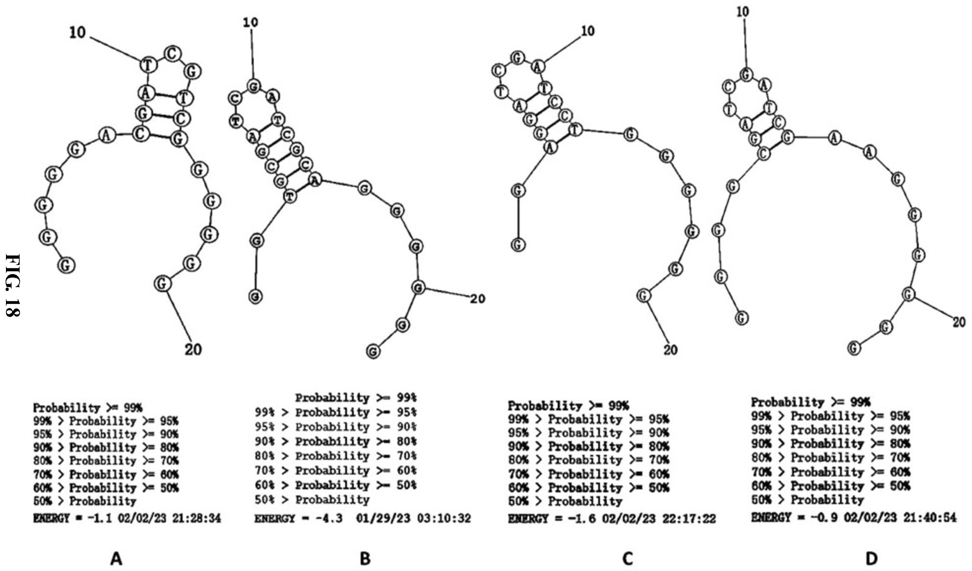

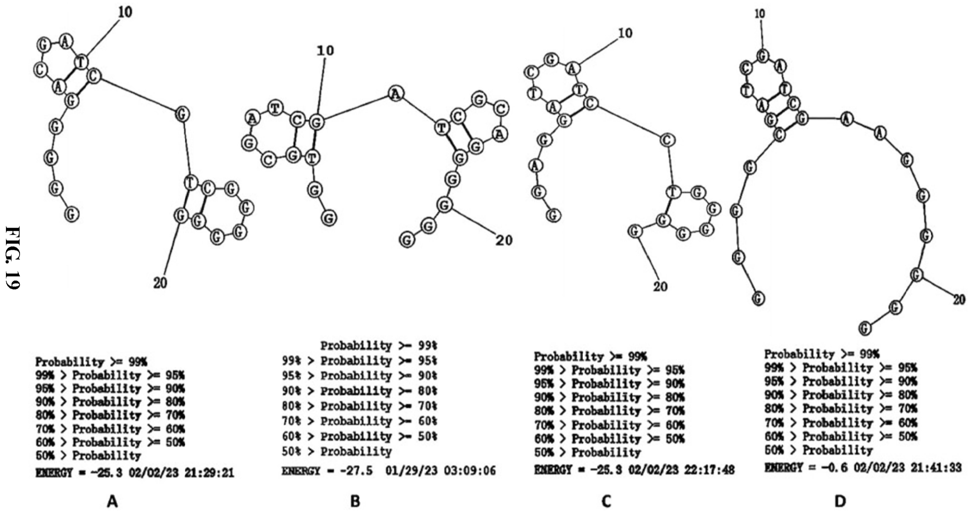

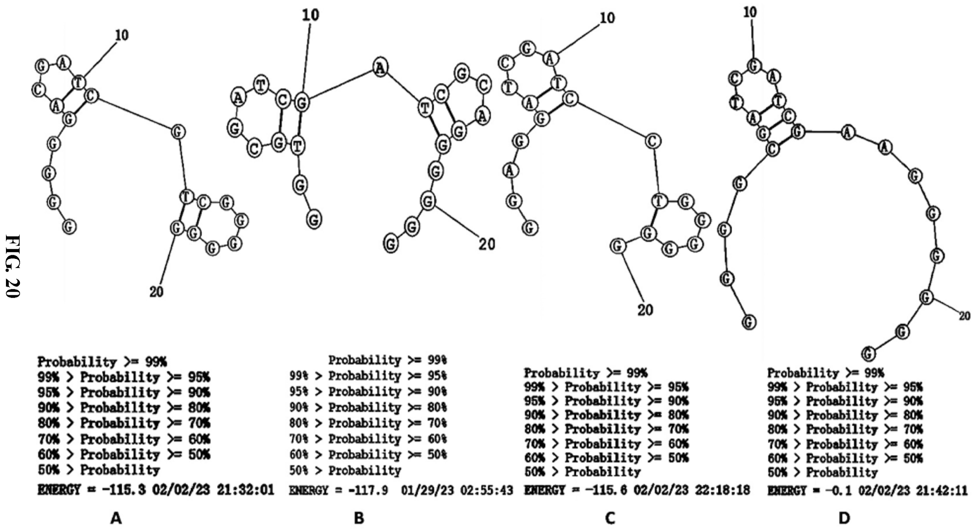

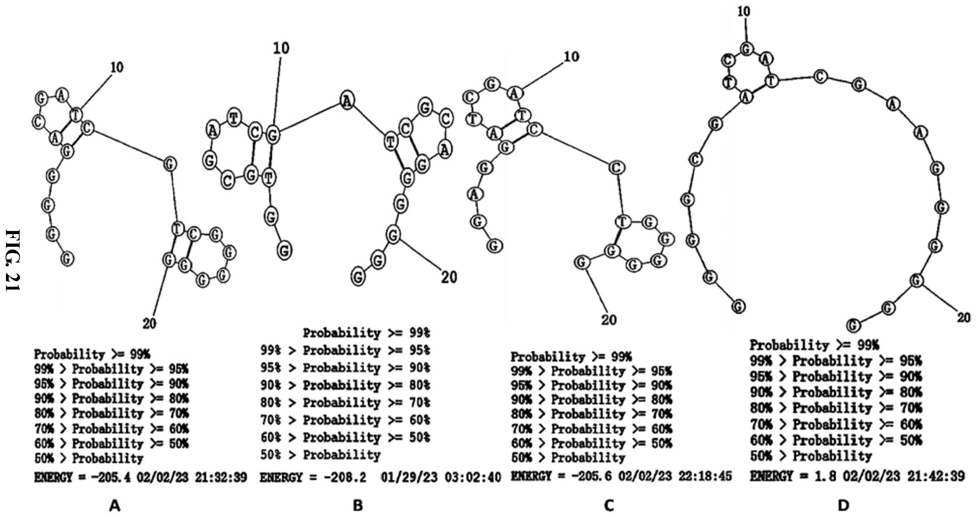

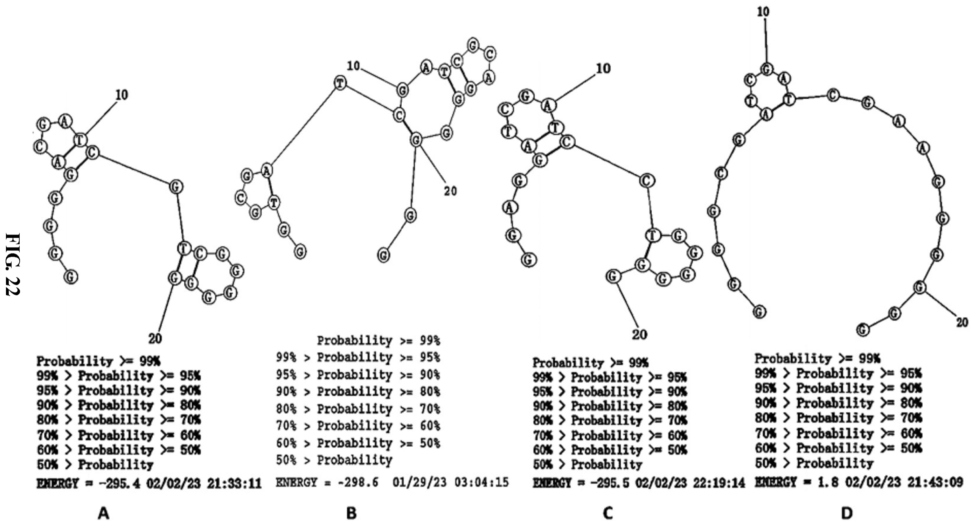

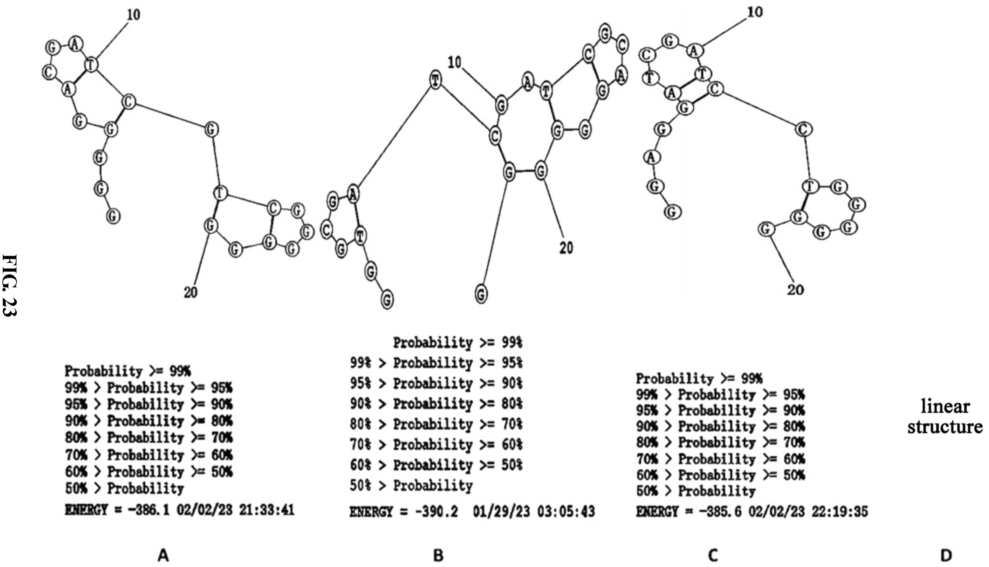

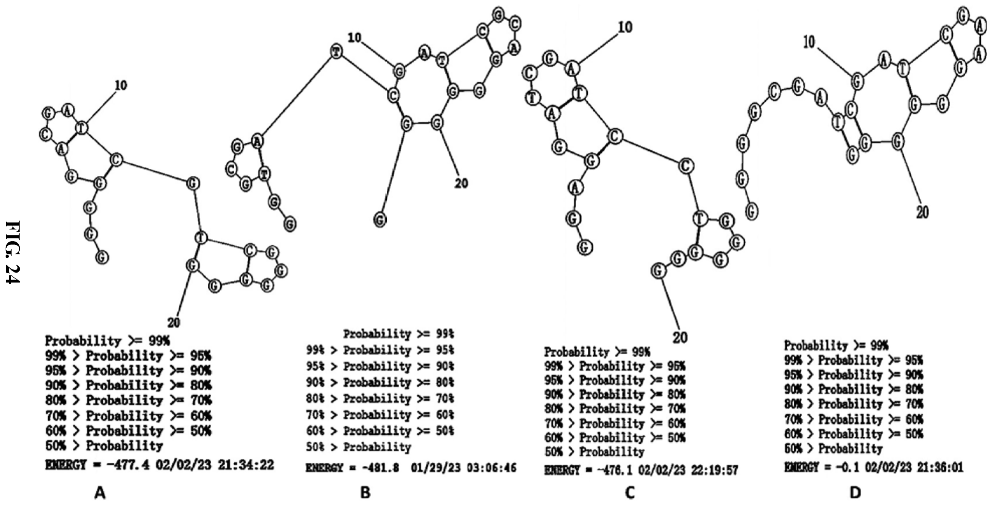

The specific secondary structure diagrams are shown in Figure 14-24. Comparing the secondary structures of the four compounds at different temperatures, it can be seen that CpG 2216 is relatively stable between 4°C and 37°C. When the temperature exceeds 40°C, its secondary structure undergoes misfolding, affecting its biological activity. E41-1 is relatively stable between 0°C and 37°C. When the temperature reaches -20°C, localized internal hydrogen bond breakage occurs. When the temperature exceeds 40°C, its secondary structure undergoes misfolding; both excessively higher and lower temperatures affect its biological activity. AM1012-03 is also relatively stable between 0°C and 37°C. When the temperature reaches 0°C, localized internal hydrogen bond breakage occurs. When the temperature exceeds 40°C, its secondary structure also undergoes misfolding, thus affecting its biological activity. AM1012-05 maintains its constant secondary structure between 0°C and 50°C. Only when the temperature exceeds 60°C does localized internal hydrogen bond breakage occur. This indicates that the secondary structure of the AM1012-05 sequence is more stable than that of CpG 2216, E41-1, and AM1012-03. This also explains structurally why AM1012-05 has better stability and activity than CpG 2216, E41-1, and AM1012-03.

### Example 16: Evaluation of the Stimulatory Effect of CpG ODN with Different Degrees of Multimerization on Rat Spleen Cells

(1) A certain amount of 100 µM CpG ODN solution treated under different temperatures was formulated as a stock solution of a certain concentration using RPMI 1640 complete medium. Then, the CpG ODN solution was diluted with complete medium in a 4-fold ratio to prepare test samples of different concentrations.
(2) An appropriate amount of fresh SD rat spleen cells were obtained.
(3) 1 × 10⁶ cells/well were plated in a 96-well plate, with different concentrations (final concentrations of 3.2µM and 0.8µM, respectively) of CpG ODN samples added to each well, with 3 replicates per group. PBS blank control wells were also included.
(4) The plate was incubated at 37°C in a 5% CO₂ incubator for 72 h.
(5) The supernatant was collected by centrifugation.
(6) IFN-γ was detected according to ELISA test instructions for rat IFN-γ .

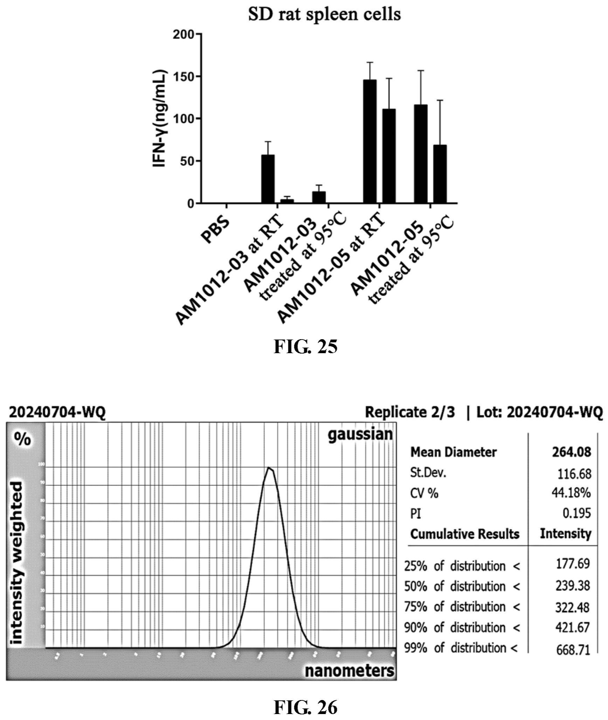

The experimental results are shown in Figure 25. The designed AM1012-05 sequence can still effectively stimulate the secretion of IFN-γ by spleen cells of SD rats after high-temperature treatment, while the AM1012-03 sequence cannot effectively stimulate the secretion of IFN-γ by spleen cells of SD rats after high-temperature treatment. Combined with the secondary structure prediction results of the sequence in Example 15, the high-temperature stability of AM1012-05 is much better than that of CpG 2216, E41 and AM1012-03. This experiment verifies that a stable secondary structure is a prerequisite for the activity of CpG.

### Example 17: Stability Variation of CpG ODNs as Raw Material under Different Preparation Processes

E41-2, E41-3, and AM1012-05 raw materials were formulated into 2 mg/mL stock solutions and subjected to different processing methods: direct lyophilization or lyophilization after heating at 95°C. Dry powders prepared by different lyophilization processes were dissolved, and their multimerization degree and related substances were analyzed by reversed-phase HPLC. The proportion of multimerized states of E41-2, E41-3, and AM1012-05 prepared by different processes varied slightly. However, the impurities detected in the dry powders of E41-2 and E41-3 prepared by different processes differed significantly. In contrast, the impurity levels detected in AM1012-05 prepared under different processes were basically the same. The results indicate that AM1012-05 has better stability than E41-2 and E41-3, and exhibits better drugability.

**Table 6. Stability analysis of CpG ODNs as raw material under different preparation processes.**

| CpG raw material | Content | Direct lyophilization | lyophilization after depolymerized at 95°C |
|---|---|---|---|
| AM1012-05 | Impurity peak ratio | 3.76% | 4.62% |
| | Active drug ratio | 91.30% | 91.40% |
| | Multimer peak ratio | 4.94% | 3.98% |
| E41-2 | New impurity peak ratio | 4.26% | 11.29% |
| | Active drug ratio | 90.42% | 83.69% |
| | Multimer peak ratio | 5.32% | 5.02% |
| E41-3 | New impurity peak ratio | 6.90% | 12.56% |
| | Active drug ratio | 90.36% | 85.14% |
| | Multimer peak ratio | 2.74% | 2.30% |

### Example 18: pH Screening of AM1012-05 Formulation

(1) Sodium phosphate buffer solutions were prepared with different pH values: pH 6.6, 7.0, 7.2, 7.4, 8.0, and 9.0.
(2) AM1012-05 was dissolved in the above buffer solutions to prepare a 0.35 mg/ml formulation solution.
(3) 1.5 mL of each of the above formulation solutions was dispensed into a 10 mL vial. Vials were stoppered and capped and stored at 50 °C in the dark.
(4) Samples were collected at 0, 5, and 10 days to detect the content.

The experimental results are shown in Table 7. The results indicate that AM1012-05 is easily degraded in a slightly acidic environment. The formulation is stable within the pH range of 7.0-9.0.

**Table 7. Screening of pH range for samples**

| pH | AM1012-05 content% (specification: 0.35 mg/mL) | | |
|---|---|---|---|
| | Day 0 | Day 5 | Day 10 |
| 6.6 | 95.74 | 84.61 | 78.36 |
| 7.0 | 94.41 | 89.36 | 87.4 |
| 7.2 | 92.78 | 89.54 | 88.8 |
| 7.4 | 94.05 | 92.42 | 91.6 |
| 8.0 | 94.31 | 93.54 | 92.9 |
| 9.0 | 102.9 | 104.8 | 101.2 |

### Example 19: Selection of Buffer Solution for AM1012-05 Solution

Based on the pH range and commonly used buffer systems of marketed small nucleic acid drugs, phosphate and citrate buffer systems were screened. 2.5 mg/ml was selected as the concentration of AM1012-05 experimental sample, with an aqueous solution of AM1012-05 without buffer as a control. The pH of the samples was controlled at 7.2. The samples were placed at 50°C for 10 days, and sampling were performed at 0, 5, and 10 days to compare whether the stability of the samples in the two buffer solutions was superior to the control group. The results showed that compared with the control group, the purity change of the sample with buffer added was significantly reduced. The trends of all target properties of the two buffer systems were consistent with the extension of storage time. The experimental results are shown in Table 8. Both buffer systems can meet the pH buffer requirements of the formulation.

**Table 8. Screening of buffers**

| Investigation items | Control | | | phosphate buffer | | | citrate buffer | | |
|---|---|---|---|---|---|---|---|---|---|
| days | 0 | 5 | 10 | 0 | 5 | 10 | 0 | 5 | 10 |
| appearance | colorless clear liquid | | | colorless clear liquid | | | colorless clear liquid | | |
| Clarity | ≤0.5 | ≤0.5 | ≤0.5 | ≤0.5 | ≤0.5 | ≤0.5 | ≤0.5 | ≤0.5 | ≤0.5 |
| pH | 7.1 | 7.1 | 7.3 | 7.2 | 7.3 | 7.3 | 7.1 | 7.2 | 7.3 |
| Purity (%) | 97.3 | 89.8 | 80.2 | 97.2 | 95.7 | 94.3 | 97.4 | 96.4 | 95.4 |

### Example 20: Screening of Metal Ion Concentration Range for CpG ODNs

5 mg each of sodium acetate, sodium dihydrogen phosphate/disodium hydrogen phosphate, magnesium chloride, calcium chloride, potassium chloride, and sodium chloride were separately formulated as 200 µl solutions with 0.25 mg AM1012-05. The test samples were placed in a sealed, clean container and incubated at 40°C for 30 days, with sampling performed on days 5, 15, and 30. The detection indicators included character (appearance), solubility of interest, impurities (related substances), and content.

The results showed that sodium acetate, sodium dihydrogen phosphate/disodium hydrogen phosphate, magnesium chloride, calcium chloride, potassium chloride, and sodium chloride precipitated when mixed with AM1012-05 at the specified concentrations. Therefore, it is necessary to screen for suitable salt concentrations for CpG ODN formulations to ensure the clarity of the formulation solution.

AM1012-05 was added to potassium chloride and sodium chloride solutions, respectively, resulting in solutions with a final AM1012-05 concentration of 2 mg/ml and a final salt solution concentration of 90 mM. After 24 hours of standing, AM1012-05 remained clear in both salt solutions.

AM1012-05 was added to magnesium chloride and calcium chloride solutions, respectively, resulting in solutions with a final AM1012-05 concentration of 2 mg/ml and a final salt solution concentration of 1.8 mM. After 24 hours of standing, AM1012-05 remained clear in both salt solutions.

Four phosphate buffer solutions (composed of sodium dihydrogen phosphate and disodium hydrogen phosphate) at concentrations of 5, 10, 20, and 50 mM were selected as the test concentrations (sodium ion concentrations of approximately 9, 18, 36, and 90 mM, respectively), with isotonic (150 mM) PBS solution (sodium ion concentration of approximately 179 mM) as control. Using 2.5 mg/ml as the experimental concentration of AM1012-05, and controlling the sample pH at 7.2, the sample was placed at 50°C for 10 days, and sampling were performed at 0, 5, and 10 days to test the stability of samples and determine the pH buffer concentration. The results are shown in Table 9. Compared with day 0, after 5 days of standing at 50°C, the clarity and appearance of the samples did not change significantly, and the purity did not differ significantly with the extension of storage period. However, AM1012-05 under isotonic PBS condition showed obvious turbidity. Therefore, the isotonic (150 mM) PBS salt concentration is not suitable for preparing AM1012-05 formulations. Considering both the buffering capacity of pH and the risk of precipitation of AM1012-05, the sodium ion concentration in the solution formulation was selected to be between 0.1 and 90 mM. The total concentration range of metal ions such as sodium, potassium, magnesium, calcium, zinc, and iron in the CpG ODN solution formulation is between 0.1 and 90 mM.

**Table 9. Screening of buffer salt concentrations**

| Lot# | Appearance | | | Clarity (Turbidity Standard Solution, Chinese Pharmacopoeia 2022 Edition) | | | Purity% | | | Multimer | | |
|---|---|---|---|---|---|---|---|---|---|---|---|---|
| | 0h | 24h | 5d | 0h | 24h | 5d | 0h | 24h | 5d | 0h | 24h | 5d |
| 5 mM Phosphate buffer | colorless clear liquid | | | Grade ≤0.5 | | | 95.51 | 95.38 | 96.55 | 2.75 | 2.38 | 0.67 |
| 10 mM Phosphate buffer | colorless clear liquid | | | Grade ≤0.5 | | | 95.23 | 95.21 | 96.51 | 2.66 | 2.39 | 0.89 |
| 20 mM Phosphate buffer | colorless clear liquid | | | Grade ≤0.5 | | | 95.28 | 95.48 | 96.43 | 2.33 | 2.27 | 0.7 |
| 50 mM Phosphate buffer | colorless clear liquid | | | Grade ≤0.5 | | | 94.45 | 94.33 | 96.25 | 3.12 | 3.28 | 0.94 |
| 150mM PBS | turbid | | | Grade >2 | | | 92.77 | 91.88 | 96.28 | 4.71 | 5.49 | 1.02 |

### Example 21: Selection of Isotonic Adjuster for AM1012-05 Solution

Sodium chloride is generally used to adjust isotonicity in solutions. However, since the formation of Type A CpG multimers is related to the concentration of metal ions in the solution, isotonic sodium chloride solutions can cause Type A CpG to precipitate out of the solution. Therefore, additional excipients are needed as isotonic adjusters.

Excipients that can be used as isotonic adjusters include glycine, trehalose, mannitol, sucrose, arginine, lysine, histidine, sorbitol, glucose, glycerol, and propylene glycol.

In each tube of sample, 0.25 mg AM1012-05 and 5 mg of the excipient said above were formulated as a 200 µl solution. The sample to be tested was placed in a sealed, clean container and incubated at 40°C for 30 days, with sampling performed on days 5, 15, and 30. Detection indicators include character(appearance), solubility, impurities (related substances), and content. The test results showed that the solutions of sorbitol and glucose mixed with AM1012-05 exhibited abnormal peak shapes in the HPLC chromatogram, indicating poor compatibility between sorbitol, glucose, and AM1012-05, making them unsuitable as isotonic adjusters in AM1012-05 formulations. Glycine, trehalose, mannitol, sucrose, arginine, lysine, histidine, glycerol, and propylene glycol are compatible with AM1012-05 and can be used as isotonic adjusters for AM1012-05.

AM1012-05 was formulated as a 0.35 mg/ml solution with three isotonic adjusters and phosphate buffer. The appearance, clarity, pH, osmotic pressure, and purity were tested. The specific formulation is shown in Table 10.

**Table 10. AM1012-05 formulation**

| Formulation | Formulation 1 | Formulation 2 | Formulation 3 |
|---|---|---|---|
| Isotonic adjuster | Mannitol 5% | Glycerin 2.3% | Propylene glycol 2% |

The experimental results are shown in Table 11. The results show that all three formulations of AM1012-05 can provide clear solutions, with pH, osmotic pressure and purity meeting the formulation requirements.

**Table 11. Result detected from 3 AM1012-05 formulations**

| Adjustors | Mannitol | Glycerin | Propylene glycol |
|---|---|---|---|
| Appearance | Colorless clear liquid | Colorless clear liquid | Colorless clear liquid |
| Clarity | ≤0.5 | ≤0.5 | ≤0.5 |
| pH | 7.33 | 7.27 | 7.31 |
| Osmotic pressure, Osmol/kg | 324 | 284 | 296 |
| Purity% | 96.4 | 96.33 | 96.4 |

### Example 22: Development of AM1012-05 Lyophilization Formulation

AM1012-05 was dissolved in deionized water, and mannitol (lyophilization protectant) and pH adjuster (disodium hydrogen phosphate + citric acid) were added. A solution containing 1 mg/mL AM1012-05, 5% mannitol, and 50 mM disodium hydrogen phosphate + citric acid was prepared.

0.2 mL of the above solution was filled into 2 mL vial, partially stoppered, and transferred to a lyophilizer and lyophilized at -30°C. After lyophilization, the sample was stoppered and capped to obtain a white lyophilized powder cake. This lyophilized formulation was immediately reconstituted upon addition of 1 mL of water for injection. The lyophilized formulation of AM1012-05 can preserve AM1012-05 in a solid form, which is beneficial to the stability of the active drug and is a candidate formulation for the liquid formulation of AM1012-05.

### Example 23: Selection of Other Excipients for AM1012-05 Solution Formulation

In CpG ODN solution formulations, in addition to isotonic adjusters and pH buffer systems, other excipients can be added to provide corresponding functions.

Other optional excipients include surfactants (such as Pluronic F127, Pluronic F68, Tween 20, and Tween 80), preservatives (such as benzalkonium chloride), metal chelating agents (disodium edetate), viscosity modifiers (hydroxypropyl methylcellulose, sodium carboxymethyl cellulose citrate), methyl beta-cyclodextrin, hydroxypropyl beta-cyclodextrin, antioxidants (ascorbic acid), etc.

In this experiment, 5 mg of Pluronic F127, Pluronic F68, Tween 20, Tween 80, and ascorbic acid were respectively mixed with 0.25 mg of AM1012-05 to prepare 200 µl solution samples. The test sample was placed in a sealed, clean container and incubated at 40°C for 30 days. Sampling were performed on days 5, 15, and 30. Detection indicators include character(appearance), solubility of interest, impurities (related substances), and content.

Meanwhile, a 0.4 mg/mL formulation solution was prepared by adding AM1012-05 to a solution of benzalkonium chloride, disodium edetate, sodium citrate, hydroxypropyl methylcellulose, sodium carboxymethylcellulose, methyl beta-cyclodextrin, and hydroxypropyl beta-cyclodextrin prepared according to the concentrations in Table 12, with a pH of 7.0-7.5. The test sample was placed in a sealed, clean container and incubated at 40°C for 30 days, sampling performed on days 5, 15, and 30. Detection indicators include character(appearance), solubility of interest, impurities (related substances), and content.

**Table 12. Listing of Excipients**

| **Excipients** | **Target concentration** |
|---|---|
| Benzalkonium chloride (7.0-7.5) | 0.0014 mM |
| Disodium edetate (7.0-7.5) | 11.90mM |
| Sodium citrate (7.0-7.5) | 9.8mM |
| Sodium hydroxypropyl methylcellulose/carboxymethyl cellulose (7.0-7.5) | 0.83mg/ml (each) |
| Methyl beta-cyclodextrin | 64mM |
| Hydroxypropyl beta-cyclodextrin(7.0-7.5) | 64mM |

The test results showed that mixing ascorbic acid with AM1012-05 would cause AM1012-05 degradation, therefore it is not suitable for use in AM1012-05 formulations. HPLC analysis of a mixture liquid of hydroxypropyl beta-cyclodextrin and AM1012-05 showed abnormal peak shapes for the corresponding AM1012-05 main peak, therefore it is also unsuitable for AM1012-05 formulations.

Pluronic F127, Pluronic F68, Tween 20, Tween 80, benzalkonium chloride, disodium edetate, sodium citrate, hydroxypropyl methylcellulose, sodium carboxymethylcellulose, and methyl beta-cyclodextrin are compatible with AM1012-05 and can be used as excipients for AM1012-05-related formulations.

### Example 24: Preparation of PLGA nanoformulations with CpG ODNs

(1) 100 mg PLGA was dissolved in 1.25 mL dichloromethane (oil phase).
(2) A solution of 12 mg/mL AM1012-05 and 1% PVA (aqueous phase 1) was prepared.
(3) 4 mL of 2.5% PVA solution (aqueous phase 2) was prepared.
(4) The oil phase and aqueous phase 1 were homogenized to form a primary emulsion. Then the primary emulsion was added to the aqueous phase 2 and the mixture was homogenized using a homogenizer to form a secondary emulsion.
(5) The formed secondary emulsion was slowly added to a 1% PVA aqueous solution (external aqueous phase) and stirred for 4 hours to allow the dichloromethane to evaporate.
(6) The solution from step 5 was centrifuged at 500 g for 5 minutes to remove large microspheres and collect the supernatant.
(7) The supernatant from step 6 was centrifuged at 10000 g for 10 minutes. The solid was collected and washed three times with deionized water or ultrapure water to obtain the PLGA microsphere product.

The obtained PLGA microspheres were tested by a Nicomp Z3000 laser particle size analyzer, and the particle size could be controlled within 500 nanometers (as shown in Figure 26).

### Example 25: Preparation of Chitosan Nanoparticles with CpG ODNs

(1) A 0.5 mg/ml chitosan (molecular weight 200,000) solution was prepared, with pH adjusted to 5.6.
(2) A 50 µg/ml AM1012-05 solution was prepared.
(3) 10 ml of AM1012-05 solution was added dropwise to 10 ml of chitosan solution and stirred for 1 minute.

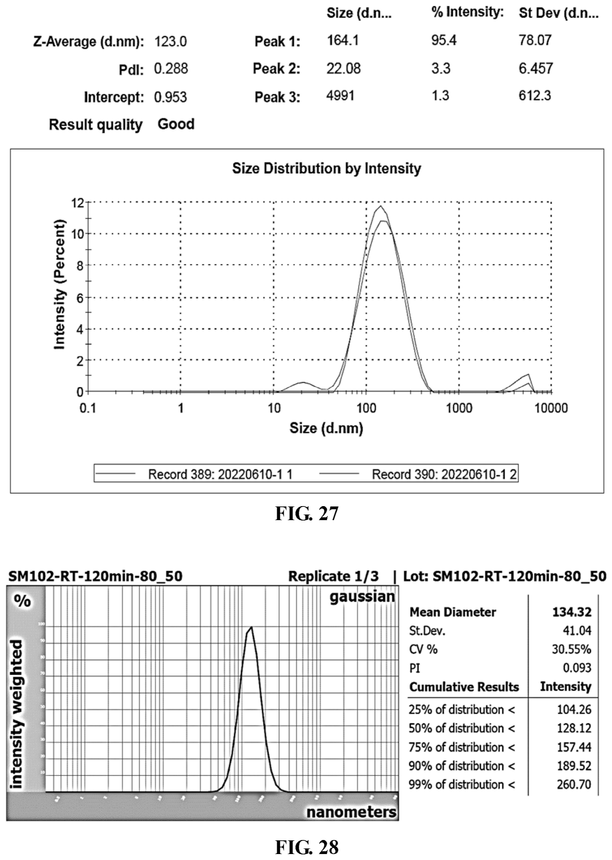

The AM1012-05 chitosan nanoparticles prepared in this experiment were tested by a laser particle size analyzer and dynamic light scattering particle size analyzer. The particle size could be controlled to be around 150 nm (as shown in Figure 27).

### Example 26: Preparation of Lipid Nanoparticles with CpG ODNs

Lipid nanoparticles (LNPs) of CpG ODNs were synthesized by self-assembling by selecting different types of lipids and mixing them in a certain proportion with CpGs. Lipids included ionizable cationic liposomes, neutral phospholipids, sterol liposomes, and PEGylated phospholipids. The specific preparation process was as follows:
(1) SM-102, DSPC, cholesterol, and DMG-PEG(2000) were dissolved in ethanol at a ratio of 50:10:38.5:1.5.
(2) 0.25 ml of the above lipid solution was added to 0.75 ml of 80 µg/ml AM1012-05 in 50 mM citrate buffer solution. After vortex mixing, the mixture was transferred to a hand-operated liposome extruder.
(3) Further mixing was performed by rapidly pipetting through the extruder. The mixture was allowed to stand for 10 minutes.
(4) Using a 200 kDa dialysis bag, an equal volume of 50 mM citrate buffer solution was added to the mixture. After sealing, the bag was placed in 50 ml of PBS solution for dialysis.

The lipid nanoparticles of AM1012-05 prepared in this experiment were tested using a Nicomp Z3000 laser particle size analyzer, and the particle size could be controlled to approximately 100 nanometers (as shown in Figure 28).

### Example 27: Preparation of PLGA Nanoparticles with CpG ODNs as an Immunoadjuvant

(1) 100 mg PLGA was dissolved in 1.25 mL dichloromethane (oil phase).
(2) AM1012-05 and antigen protein (S1 protein) were added into a 1% PVA solution to prepare a solution containing 10 mg/mL AM1012-05 and 10 mg/mL S1 protein. (aqueous phase 1)
(3) 4 mL of 2.5% PVA solution was prepared (aqueous phase 2).
(4) The oil phase and aqueous phase 1 were homogenized to form a primary emulsion. Then the primary emulsion was added to the aqueous phase 2 and homogenized using a homogenizer to form a secondary emulsion.
(5) The formed secondary emulsion was slowly added to a 1% PVA aqueous solution (external aqueous phase) and stirred for 4 h to allow the dichloromethane to evaporate.
(6) The solution from step 5 was centrifuged at 500 g for 5 minutes to remove large microspheres and collect the supernatant.
(7) The supernatant from step 6 was centrifuged at 10000×g for 10 minutes. The solid was collected and washed three times with deionized water or ultrapure water to obtain the PLGA microsphere product.

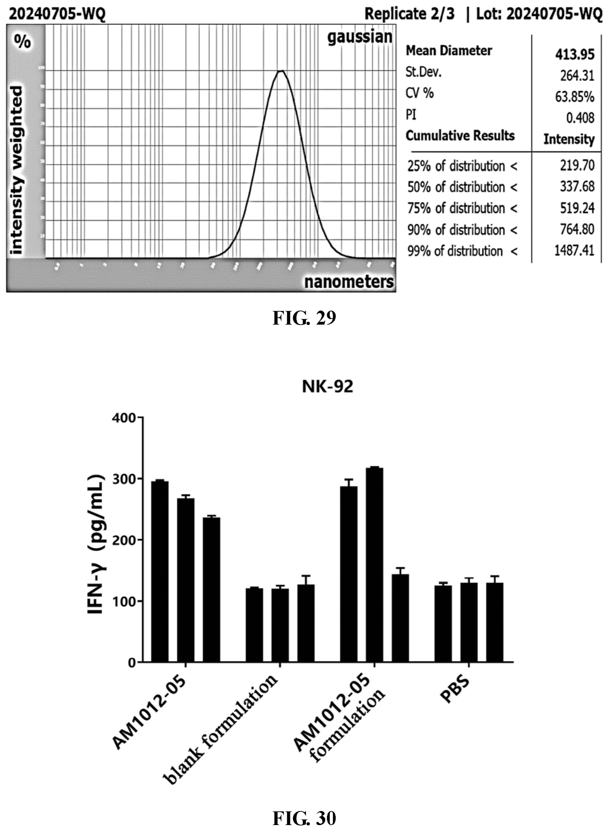

The PLGA microsphere vaccine product encapsulating AM1012-05 and the antigen protein obtained in this experiment was tested using a Nicomp Z3000 laser particle size analyzer, and the particle size could be controlled within 500 nanometers (as shown in Figure 29).

### Example 28: Compatibility Study of Excipients in Topical Formulations of CpG ODNs

Excipients that can be used in topical formulations are classified as oil phase, thickeners, gelling agents, emulsifiers, solvents, and penetration enhancers. The oil phase matrix includes glyceryl behenate, liquid paraffin, petrolatum, medium-chain triglycerides, and cocoyl octanoate/decanoate. The thickeners include glyceryl monostearate, glyceryl mono- and di-stearates, and cetearyl alcohol. The gelling agents include hydroxypropyl methylcellulose, xanthan gum, carbomer, sodium carboxymethyl cellulose, and poloxamer 407. The emulsifiers include polyoxyl 40 stearate, polyethylene glycol cetostearyl ether, Tween 60, poloxamer 188, oleoyl polyoxylglycerides, PEG-7 stearate, Labrasol, sodium lauryl sulfate, propylene glycol monofatty acid ester, polyglycerol oleate, and propylene glycol laurate. The solvents include glyceryl triacetate and propylene glycol. The penetration enhancers include isopropyl myristate, propylene glycol, Labrasol, polyglycerol oleate, and propylene glycol laurate.

After preparing an aqueous solution of appropriate concentration of the gelling excipient, it was mixed with AM1012-05 to form a 3 mg/g gelling solution. Other excipients were also mixed with API one-to-one to form a 3 mg/g mixture. The samples were placed in a 40°C incubator, and sampling were performed at 5, 15, and 30 days to detect the content and related substances. The results showed that the excipients compatible with AM1012-05 included: oil phase matrix such as glyceryl behenate, liquid paraffin, petrolatum, medium-chain triglycerides, and cocoyl octanoate/decanoate; thickeners such as glyceryl mono- and di-stearates; gelling agents such as hydroxypropyl methylcellulose, carbomer, xanthan gum, sodium carboxymethyl cellulose, and poloxamer 407; emulsifiers such as polyethylene glycol cetostearyl ether, poloxamer 188, oleoyl polyoxylglycerides, PEG-7 stearate, PEG-7 caprylate, sodium lauryl sulfate, propylene glycol monofatty acid esters, polyglycerol oleate, and propylene glycol laurate; solvents such as propylene glycol; and penetration enhancers such as isopropyl myristate, propylene glycol, and polyglycerol oleate.

### Example 29: Preparation of a Topical Ointment Formulation of CpG ODNs

A certain amount of AM1012-05 was dissolved in deionized water to prepare an API solution. Appropriate amounts of petrolatum and liquid paraffin were heated in an 80°C water bath until they melt. The API solution was added to the molten oil phase, homogenized at 70-80°C for 10 minutes, and then cooled to 65-70°C for dispensing. The final formulation contained 1 mg/g of AM1012-05. The ratio of petrolatum to liquid paraffin was determined based on sensory evaluation; the proportion of petrolatum can be selected between 50% and 100%.

### Example 30: Preparation of a Topical Gel Formulation of CpG ODNs

An aqueous solution of carbomer 974 (2%), a solution of 10% triethanolamine, and an aqueous solution of AM1012-05 were prepared. A 2% carbomer 974 aqueous solution was diluted to a concentration of 0.7%, with pH adjusted to 7.0 with 10% triethanolamine. Phenoxyethanol was dissolved in propylene glycol, added to the gel, and mixed by stirring. Then, API aqueous solution was added and mixed by stirring 1. Later, 10% triethanolamine was added again to adjust to the appropriate pH. The resulted mixture was adjusted to a targeted volume, mixed by stirring, and dispensed.

The concentration and type of carbomer used to prepare the gel can be adjusted according to viscosity requirements, such as carbomer 971, 980, and 981. Neutralizing agents, besides triethanolamine solution, can also include sodium hydroxide, aminomethylpropanol, aminobutanetriol, tetra-2-hydroxypropylethylenediamine, etc. Preservatives, besides phenoxyethanol, can also include parabens and cresols.

### Example 31: Preparation of Topical Emulsion of CpG ODNs

(1) Oil Phase: 15g of petrolatum, 6g of liquid paraffin, and 7.2g of glyceryl mono-and di-stearates were placed in a container and heated to 70°C, stirring until melted. The molten oil phase was maintained at 70°C and stirred at low speed.
(2) Aqueous Phase: 59.5g of deionized water was heated to 70°C in a container, and 1.8g of polyethylene glycol cetostearyl ether was added and dissolved.
(3) Emulsion Preparation: At 70°C, the molten oil phase from step 1 was transferred to the aqueous phase container, while mixing at 10 rpm.
(4) After the transfer, a homogenizer was started, and homogenization was performed for 10 minutes, with the temperature controlled between 65°C and 70°C.
(5) The homogenizer was stopped, and a stirrer was set to 50°C to cool the emulsion to 50°C.
(6) Drug Solution: 30 mg of AM1012-05 was dissolved in 10.5 g of deionized water, maintaining the temperature at 50°C.
(7) The drug solution from step 6 was transferred to the emulsion from step 5, stirring at 50°C.
(8) The homogenizer started to mix and homogenize the resultant from (7) for 10 minutes, while adding 10% sodium hydroxide solution to adjust the pH to 7.5.
(9) When the emulsion cools to 25°C, the prepared emulsion was dispensed, finally obtaining 100 g of 0.3 mg/g AM1012-05-containing emulsion.

The oil phase, thickener, and emulsifier used in this emulsion can be replaced with excipients that have passed the excipient compatibility test. Penetration enhancer excipients that have passed the excipient compatibility test can also be added to the emulsion.

### Example 32: Preparation of a Vaccine Using CpG as an Adjuvant

This example uses the SARS-CoV-2 S1 protein as a model antigen and CpG as an adjuvant to prepare a vaccine.
(1) Under aseptic conditions, 50 µL of SARS-CoV-2 S1 protein (2 mg/ml PBS solution) was adjusted a volume of 250 µL with PBS.
(2) A 2 mg/mL AM1012-05 or CpG 1018 solution was prepared.
(3) 250 µL of the solution from step (2) was added to the solution from step (1) and mixed thoroughly.

### Example 33: Preparation of Vaccines Using Adjuvant Systems (Aluminum Adjuvant + CpG)

This example uses the SARS-CoV-2 S1 protein as a model antigen to prepare a vaccine using an adjuvant system (aluminum adjuvant + CpG).

### Preparation of a vaccine using aluminum hydroxide + CpG as an adjuvant:

(1) 50 µL of SARS-CoV-2 S1 protein (2 mg/ml PBS solution) was prepared.
(2) 50 µL of aluminum hydroxide gel adjuvant (2%) was added to the solution from step (1).
(3) The solution from step (2) was added to 350 µL PBS solution and mixed thoroughly.
(4) 50 µL of AM1012-05 solution with a concentration of 2 mg/mL was added to the above solution and mixed evenly to allow the adjuvant to effectively adsorb the antigen.

### Preparation of a vaccine using aluminum phosphate + CpG as an adjuvant:

(1) 50 µL of SARS-CoV-2 S1 protein (2 mg/ml PBS solution) was prepared.
(2) 100 µL of aluminum phosphate gel adjuvant (0.5%) was added to the solution from step (1).
(3) The solution from step (2) was added to 300 µL PBS solution and mixed thoroughly.
(4) 50 µL of AM1012-05 solution with a concentration of 2 mg/mL was added to the above solution and mixed evenly to allow the adjuvant to effectively adsorb the antigen.

### Example 34: Effects of AM1012-05 Solution Formulation on IFN-γ Release From NK-92 Cells

(1) NK-92 cells were prepared to the required cell volume as described in Example 3, and resuspended in a certain amount of complete culture medium.
(2) 20 µL of cell suspension was mixed with trypan blue at a 1:1 ratio and counted using a cell counter to determine cell viability and density.
(3) A certain amount of AM1012-05 solution formulation sample was formulated as a stock solution using complete medium. Then, the AM1012-05 formulation solution was diluted with complete medium in a 4-fold ratio to prepare test AM1012-05 formulation samples of different concentrations.
(4) 2.5 × 10⁵ cells/well were plated in a 96-well plate, with different concentrations (final concentrations of 3.2µM, 0.8µM, and 0.2µM, respectively) of AM1012-05 samples added to each well, with 3 replicates per group. PBS blank control wells were also included.
(5) The cells were incubated at 37°C in a 5% CO₂ incubator for 72 hours.
(6) After cell culture, the cells were centrifuged at 1200 rpm for 5 min, and 100 µL of cell-free supernatant was collected; the secretion of IFN-γ in the supernatant was detected according to the ELISA kit instructions.

The experimental results are shown in Figure 30. The stimulatory effect of the AM1012-05 solution formulation samples on the secretion of IFN-γ from NK cells is comparable to that of AM1012-05, and the blank formulation sample does not stimulate NK-92 cells. This indicates that the components of the solution formulation used in the application has good compatibility with AM1012-05 and does not affect the immune cell activation function of AM1012-05.

### Example 35: Effects of AM1012-05 on the Immune System of C57BL6 Mice After Single Nasal Administration

The immune activation effect of AM1012-05 on the respiratory tract of C57BL6 mice was assessed by intranasal administration. They were divided into two groups in the experiment: the dosage group received 100 µg of AM1012-05 per mouse (25 µL/nostril, dissolved in PBS), and the negative control group received 50 µL of PBS (25 µL/nostril). The experiment ended 24 hours after administration, and samples (lungs and spleen) were collected. Changes in various immune indicators were analyzed by RT-qPCR.

The experimental results are shown in Figure 31. The figure shows that AM1012-05 effectively stimulated the expression of IFN-α, IFN-γ, and CXCL-10 in the lungs and spleen of the mice. Furthermore, the activation of cytokines by AM1012-05 in the spleen was far less significant than in the lungs (IFN-α and IFN-γ were approximately 4% of those in the lungs; CXCL-10 was approximately 1.5% of those in the lungs). This is likely because this experiment involved intranasal administration, meaning only a very small amount of AM1012-05 entered the spleen cells via blood circulation. This result indirectly reflects the very low systemic bioavailability of AM1012-05 after intranasal administration. Meanwhile, a dose of 100 µg/mouse did not produce significant toxic side effects in the mice.

### Example 36: Observation of Respiratory Immune biomarkers After Nasal Administration of AM1012-05 in Mice

In C57BL6 mice, the immune activation ability of AM1012-5 at different dosages and time points was assessed by intranasal administration. Three dosage groups were administered AM1012-05 at doses of 10 µg, 50 µg, and 100 µg per mouse (25 µL/nostril, dissolved in PBS). The negative control group received 50 µL PBS (25 µL/nostril), and the positive control group received 50 µg CpG 2216 (25 µL/nostril). Lung samples were collected before administration, 3 h after administration, 12 h after administration, 24 h after administration, and 48 h after administration. Changes in CXCL-10 mRNA levels were analyzed by RT-qPCR.

The results are shown in Figure 32. The figure shows that AM1012-05 effectively stimulated CXCL-10 expression in the mouse lungs. Furthermore, a dosage of 100 µg/mouse demonstrated good immunostimulatory activity, with the highest mRNA transcription level of CXCL-10 reaching 24 h post-administration. Compared to the CpG 2216 sequence (CpG 2216 is the commonly used Type A CpG in literatures), AM1012-05 exhibited better immune activation effects.

### Example 37: Observation of Respiratory Immune Biomarkers After Nasal Administration of AM1012-05 in Mice (Different Administration Frequencies)

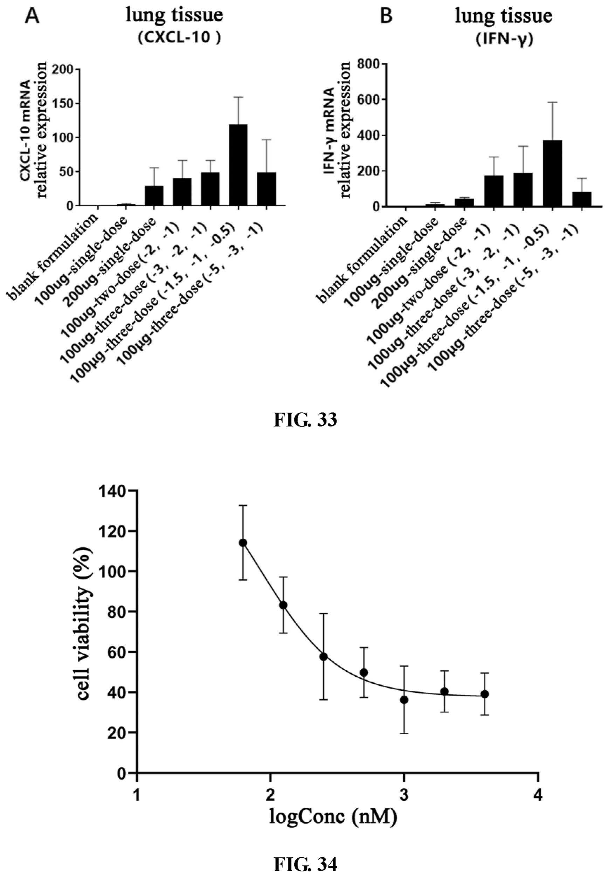

In C57BL6 mice, the immune activation capacity of AM1012-05 at different administration frequencies was evaluated by intranasal administration. The animal manipulation and biological sample detection methods were the same as in Example 36. The results are shown in Figure 33 (the numbers in parentheses on the horizontal axis represent the timepoint of each administration, with day 0 being the end of the experiment). The figure shows that AM1012-05 effectively stimulated the expression of CXCL-10 and IFN-γ in the mouse lungs. Different stimulating effects were observed at different administration frequency; with increasing administration frequency, the mRNA levels of cytokines increased to some extent. Furthermore, the administration interval significantly affected the immune activation effect, with the most significant stimulation occurring at a 12-hour administration interval.

### Example 38: Evaluation of in vitro Antitumor Activity of AM1012-05

(1) MDA-MB-231 and N87 cells were cultured and amplified, and the cells were harvested and resuspended in 2 mL of complete culture medium.
(2) 10 µL of cell suspension was mixed with trypan blue at a 1:1 ratio, and counted using a cell counter. The cell viability was 99.7% for MDA-MB-231; 100% for N87; and 99.2% for PBMCs. The cell density was 1.49 × 10⁶ cells/mL for MDA-MB-231, 1.12 × 10⁷ cells/mL for N87, and 5.17 × 10⁶ cells/mL for human PBMCs.
(3) The cells were resuspended in culture medium. MDA-MB-231 cells were plated at 2000 cells/well and N87 cells at 4000 cells/well in a 96-well plate.
(4) After complete cell adhesion, PBMCs were plated at an effector-to-target ratio of 10:1.
(5) AM1012-05 concentrations were set at 62.5 nM, 125 nM, 250 nM, 500 nM, 1000 nM, 2000 nM, 4000 nM, and 8000 nM, with six replicates for each concentration.
(6) After culturing for 5 days, CCK8 assay was performed.

The experimental results are shown in Figures 34 and 35. It can be seen from the figures that AM1012-05 effectively stimulates human PBMCs to produce a killing effect on tumor cells, and this effect is significantly dose-dependent. Based on the fact that TLR-9 is mainly found in human pDCs and B cells, and that Type A CPG primarily stimulates pDCs, this result indicates that AM1012-05 enhances the killing ability of NK cells and CD8⁺ T cells against tumor cells by stimulating pDCs.

### Example 39: Evaluation of Antitumor Activity of AM1012-05 Combined with CAR-T Therapy

A certain proportion of CAR-T cells and tumor cells were co-cultured, and a specific concentration of AM1012-05 was added thereto. They were co-incubated for 3-5 days, and then free CAR-T cells and tumor cell debris were removed by changing the medium. A certain amount of CCK8 reagent was added, and the activity of the remaining tumor cells was analyzed. The tumor cell killing rate of each group was calculated, using an untreated group and a CAR-T group as controls. The experimental results show that AM1012-05 can effectively enhance the killing effect of CAR-T cells on tumor cells in a good dose-dependent manner. This demonstrates the synergistic effect of AM1012-05 with CAR-T therapy.

### Example 40: Evaluation of Anti-Allergic Rhinitis Activity of AM1012-05

(1) Basic sensitization of BALB/c mice was performed by using ovalbumin (OVA): 25 µg OVA and 2 mg Al(OH)₃ dissolved in 100 µL of physiological saline were injected intraperitoneally into mice on Day 0, 7, and 14, respectively.
(2) Nasal stimulation: 20 µL of physiological saline containing 200 µg OVA was instilled into the nasal cavity of each mouse. Stimulation timepoints: Day 21, Day 22, Day 23, Day 28, Day 29, and Day 30.
(3) 50 µg of AM1012-05 (IAMA-001) was administered nasally 1 hour after each stimulation.
(4) From the start of the experiment, the clinical condition of the animals was observed daily until euthanasia, including mental state, activity level, diet, and water intake. Each mouse was observed for 10 minutes after the stimulation, and the number of times it scratched its nose was recorded. Nasal mucosa, nasal lavage fluid, and serum were also collected for eosinophil analysis, nasal lavage fluid immune cell analysis, and analysis of immune markers (IFN-γ, IL-4), respectively.

The experimental results are shown in Figures 36 and 37. Compared with the model group, AM1012-05 (IAMA-001 in the Figures) administration effectively reduced the number of sneezes (Figure 36, Panel A), reduced the number of nose scratches (Figure 36, Panel B), inhibited rhinorrhea (Figure 36, Panel C), and reduced the accumulation of eosinophils in the nasal mucosa (Figure 36, Panel D). It also effectively reduced the IL-4 content (Figure 36, Panel E) and increased IFN-γ expression (Figure 36, Panel F) in the serum of allergic rhinitis mice. Simultaneously, AM1012-05 reduced the infiltration of neutrophils (Figure 37, Panel A) and basophils (Figure 37, Panel B) in the nasal mucosa. These results indicate that AM1012-05 can effectively shift the body's immune response towards the Th1 type, suppressing Th2 and Th17 immune responses (a decrease in eosinophils and basophils indicates a reduced Th2 immune response, while a decrease in neutrophils indicates a reduced Th17 immune response), thereby achieving the goal of treating allergic rhinitis.

Furthermore, this experiment verified in mice that IAMA-001 (AM1012-05) has the function of reducing eosinophil count, demonstrating its potential for application in the treatment of eosinophil-related diseases.

### Example 41: Evaluation of Anti-Respiratory Syncytial Virus (RSV) Activity of AM1012-05

Experiment 1: Vero-E6 cells were plated in a 24-well plate at a density of 2 × 10⁵ cells/mL and cultured for 12-16 h until confluence reached 80%. Then, 1000 µL of the supernatants from co-incubating 1X and 5X diluted AM1012-05s with human PBMCs were added to the Vero-E6 cells and incubated for 24 h. The cells were then infected with RSVs at an MOI of 0.1 for 48 h, and samples were collected to determine viral replication in each well.

Experiment 2: Vero-E6 cells were plated in a 24-well plate at a density of 2 × 10⁵ cells/mL and cultured for 12-16 h until confluence reached 80%. Then, AM1012-05 (final concentration: 1 µM) and PBMCs (2 × 10⁶ cells/well) were added to the 24-well plate. The cells were then infected with respiratory syncytial viruses (RSVs) at an MOI of 0.1 for 48 hours, and samples were collected to determine viral replication in each well.

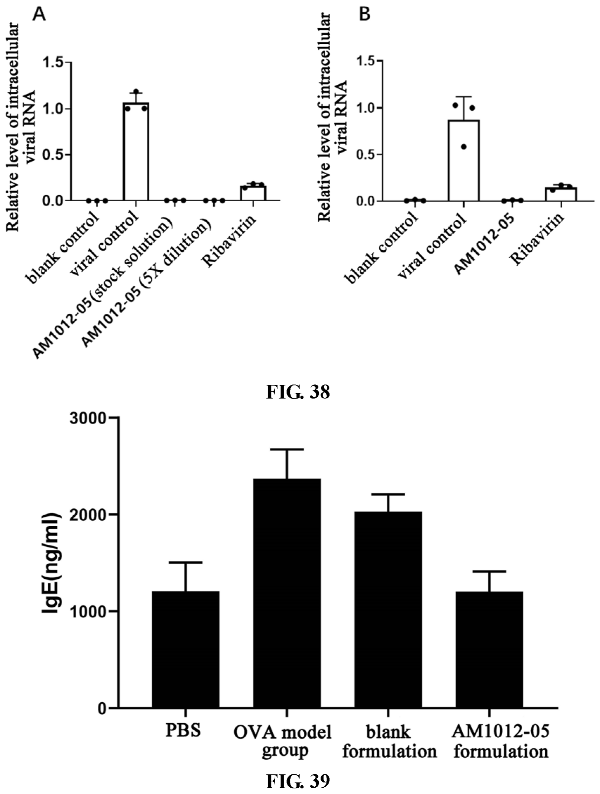

The results of the two experiments are shown in Figure 38. The supernatant of co-incubated AM1012-05 with PBMCs effectively prevented RSV proliferation in cells (Panel A), and AM1012-05 significantly cleared RSVs from host cells in the presence of PBMCs (Panel B).

### Example 42: Evaluation of Anti-Atopic Dermatitis Activity of AM1012-05's

(1) BALB/c was sensitized by injecting 0.5 mL of ovalbumin (OVA) solution (containing 5% aluminum hydroxide) on Day 0, Day 7, and Day 14 of the experiment.
(2) Preparation for administration: Animals were anesthetized, and the hair on their backs (3*3 cm area) was shaved and removed on Day 20.
(3) Local stimulation and drug administration: In the model group, the back skin was repeatedly stimulated with a brush to induce slight cracks on Day 21. In the PBS group, 50 µL of PBS was applied to a 2*2 cm gauze dressing, which was then applied to the back skin and secured with adhesive tape for 24 hours, changing it daily for one week (Days 21-27). This treatment was repeated for one week after a one-week interval (Days 35-41). In other groups, 50 µL of 1000 µg/mL OVA solution was mixed with the test sample or vehicle, applied to a 2*2 cm gauze dressing, which was then applied to the back skin and secured with adhesive tape for 24 hours, changing it daily for one week (Days 21-27). This treatment was repeated for one week after a one-week interval (Days 35-41). From Days 28-34, only the test sample or vehicle was applied for drug administration.
(4) On Day 35 of the experiment, mouse serum was collected for IgE detection.

The experimental results are shown in Figure 39. Compared with the OVA model group, AM1012-05 administration effectively reduced the serum IgE content in OVA-sensitized mice. This result indicates that AM1012-05 can effectively inhibit the body's allergic reaction, thereby achieving the goal of treating atopic dermatitis.

### Example 43: Mice Immunization and Potency Detection of the Vaccine

### Mice Immunization of the Vaccine:

(1) An appropriate number of Balb/c mice were prepared quarantined.
(2) The mice were divided into groups of 5 mice each, according to experimental requirements; the negative control group consisted of mice injected with S1 protein (S1 protein group).
(3) The mice were immunized intramuscularly on day 0 (first immunization time), day 14, and day 28, with 50 µL of vaccine per mouse.
(4) The clinical condition of the animals was recorded daily until euthanasia, including mental state, activity level, diet, and water intake;
(5) Sample Collection: Blood was collected on days 7, 21, 35, 49, 63, and 77. 100-150 µL of whole blood was collected from each mouse each time, centrifuged at 6000 rpm, 4°C, for 10 min, and the serum was then collected.
(6) The collected samples were frozen at -80°C for potency testing.

### Detection of mouse immune titer:

(1) Protein coating: The coating concentration of SARS-CoV-2 S1 protein was 2 µg/ml and the protein concentration was diluted. Diluted sample was added to a 96-well ELISA plate, 100 µL per well. The plate was sealed tightly with sealing film, and incubated overnight at 4°C.
(2) Plate Washing: liquid in the wells was discarded from the overnight coated 96-well plate. The plate was washed 3 times with 300 µL/well of PBS, and pat dried.
(3) Block: 300 µL of 2% BSA blocking buffer was added to each well. The plate was sealed tightly with sealing film and incubated at 37°C for 1 h.
(4) Plate Washing: After blocking, liquid in the wells was discarded. The plate was washed 3 times with 300 µL/well of PBST, and pat dried.
(5) Primary antibody incubation: 100 µL of diluted sample (serum) was added to each well. The plate was sealed tightly with sealing film and incubated at 37°C for 1 h.
(6) Plate Washing: After incubation, liquid in the wells was discarded. The plate was washed 3 times with 300 µL/well of PBST, and pat dried.
(7) Secondary antibody incubation: 100 µL of diluted secondary antibody was added to each well. The plate was sealed tightly with sealing film and incubated at 37°C for 1 h.
(8) Plate Washing: After incubation, liquid in the wells was discarded. The plate was washed 5 times with 300 µL/well of PBST, and pat dried.
(9) Color development: 100 µL TMB colorimetric solution was added to each well for color development at 37°C.
(10) Termination: 50 µL ELISA stop solution was added and the edge of the ELISA plate was gently tapped for mixing.
(11) Reading: The ELISA plate was placed in a microplate reader, and the plate was read at a detection wavelength of 450 nm and a reference wavelength of 570 nm.

### Analysis and discussion of immunological results:

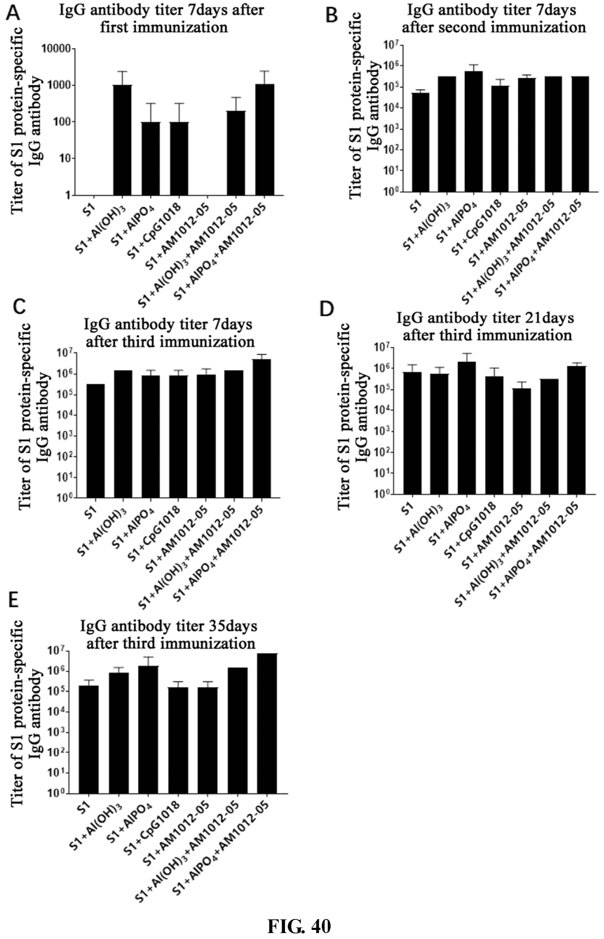

In mice, the total IgG antibody titer reflects the overall immune level of the vaccine, IgG1 reflects the humoral immune level, and IgG2a reflects the cellular immune level. Conventional adjuvants generally have good humoral immune activation ability (mouse IgG1), but weak cellular immune level (mouse IgG2a). Therefore, developing vaccine adjuvants that can effectively stimulate the production of IgG2a antibodies has been a key research focus in the adjuvant industry in recent years. Furthermore, Dynavax's CpG 1018 is currently the only CpG adjuvant approved globally for human vaccine production (CpG 1018 is used as an independent adjuvant in hepatitis B vaccine production). In this experiment, using the S1 protein of the novel coronavirus as the model antigen and CpG1018 as a CpG positive adjuvant control, the adjuvant application of the AM1012-05 vaccine prepared in Examples 32 and 33 was evaluated. The results of IgG, IgG1, and IgG2a antibody titers in mice immunized with the AM1012-05 related vaccine are shown in Figures 40, 41, and 42.

As shown in Figure 40, in the early stages of immunization, the IgG titers of the adjuvant system composed of AM1012-05 and aluminum phosphate was significantly better than that of aluminum phosphate adjuvant alone (Figure 40, Panel A); the IgG titers of the adjuvant system composed of AM1012-05 and aluminum phosphate was significantly better than that of the marketed CpG adjuvant 1018 (Figure 40, Panels A to E); the IgG titers of the adjuvant system composed of AM1012-05 and aluminum hydroxide was also better than that of the marketed CpG adjuvant 1018 (Figure 40, Panels A to E); and the IgG titers of AM1012-05 as an independent adjuvant was comparable to that of the marketed CpG adjuvant 1018 (Figure 40, Panels A to E).

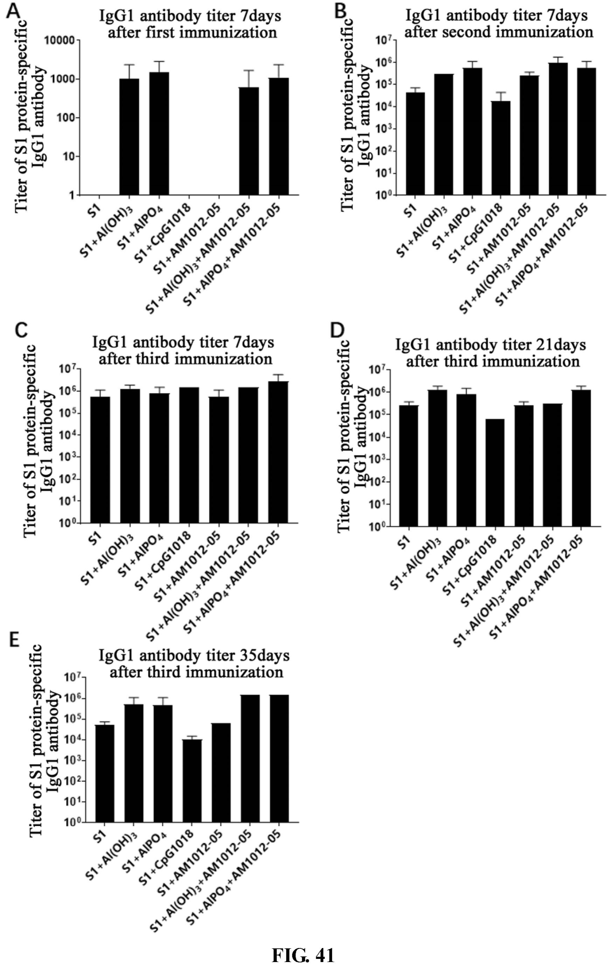

As shown in Figure 41, in the early stages of immunization, the IgG1 titers of the adjuvant system composed of AM1012-05 and either aluminum phosphate or aluminum hydroxide was significantly superior to that of the marketed CpG adjuvant 1018 (Figure 41, Panels A to B); in the middle and late stages of immunization, the IgG1 titers of the adjuvant system composed of AM1012-05 and either aluminum phosphate or aluminum hydroxide was also significantly superior to that of the marketed CpG adjuvant 1018 (Figure 41, Panels D to E); and the IgG1 titers of AM1012-05 as an independent adjuvant was comparable to that of the marketed CpG adjuvant 1018 (Figure 41, Panels A to E).

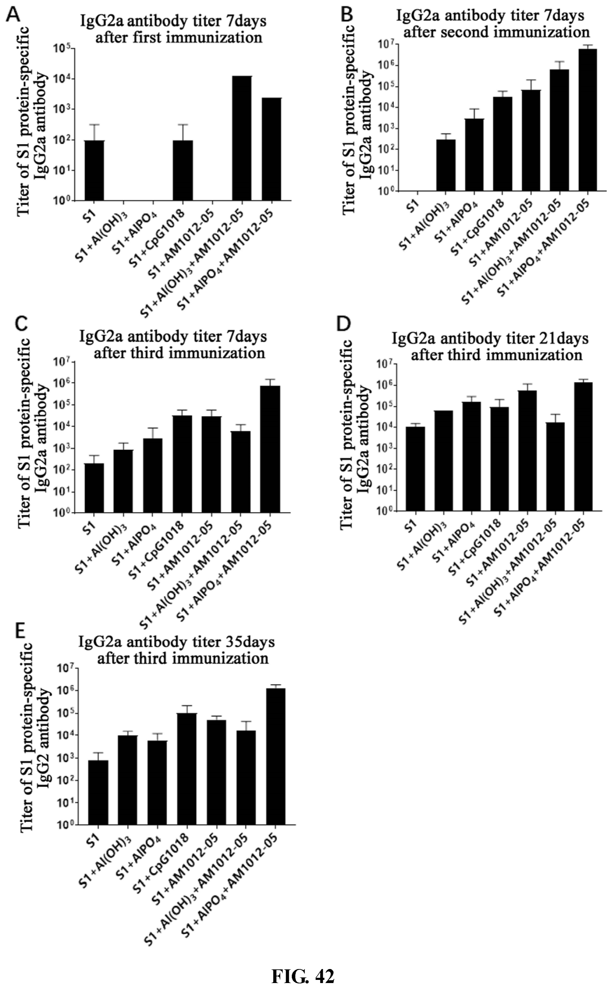

As shown in Figure 42, the IgG2a titers of the adjuvant system composed of the AM1012-05 and aluminum phosphate was significantly superior to that of the marketed CpG adjuvant 1018 (Figure 42, Panels A to E); the IgG2a titers of the adjuvant system composed of the AM1012-05 and aluminum phosphate was also significantly superior to that of the adjuvant system composed of aluminum hydroxide and aluminum phosphate (Figure 42, Panels A to E); in the early and mid-stages of immunization, the IgG2a titers of AM1012-05 as an independent adjuvant was also significantly superior to that of the marketed CpG adjuvant 1018 (Figure 42, Panels B to D); in the early stage of immunization, the IgG2a titers of the adjuvant system composed of the AM1012-05 and aluminum hydroxide was significantly superior to those of the marketed CpG adjuvant 1018, aluminum hydroxide adjuvant, and aluminum phosphate adjuvant (Figure 42, Panels A to B).

The immunogenicity evaluation (antibody titers) results of vaccines using AM1012-05 as an independent adjuvant or in adjuvant systems composed of aluminum adjuvants (aluminum hydroxide or aluminum phosphate) show that AM1012-05 significantly improves both humoral and cellular immunity levels. In particular, the adjuvant system of AM1012-05 and aluminum phosphate significantly enhances cellular immunity, with an efficacy far exceeding that of commercially available CpG adjuvant 1018 and aluminum adjuvants (aluminum hydroxide or aluminum phosphate). Furthermore, regarding early protection, the adjuvant system of AM1012-05 and aluminum adjuvants (aluminum hydroxide or aluminum phosphate) is far superior to commercially available CpG adjuvant 1018 and aluminum adjuvants (aluminum hydroxide or aluminum phosphate).

Therefore, AM1012-05, as a standalone adjuvant or in combination with aluminum adjuvants (aluminum hydroxide or aluminum phosphate), offers significant advantages over existing aluminum adjuvants (aluminum hydroxide or aluminum phosphate) and CpG adjuvants, including earlier and enhanced immunoprotective effects. It is a highly advantageous vaccine adjuvant or adjuvant system.

The present application has been described in detail above with specific embodiments and exemplary examples; however, these descriptions should not be construed as limiting the application. Those skilled in the art will understand that various equivalent substitutions, modifications, or improvements can be made to the technical solutions and implementation methods of the present disclosure without departing from the spirit and scope of the application, and all such modifications and improvements fall within the scope of the present disclosure. The scope of protection of the present disclosure is determined by the appended claims.

## Claims

1. A CpG oligodeoxynucleotide with immunomodulatory properties, comprising a sequence having the structure of 5'-(G)ₙ-core sequence-(N)_{q}-(G)m-3', wherein the core sequence is a palindromic sequence, N is any one selected from the group consisting of adenine deoxyribonucleotide (A), thymine deoxyribonucleotide (T), and cytosine deoxyribonucleotide (C); G is guanine deoxyribonucleotide; q, n, and m are all integers, in which n ranges from 2 to 11, q ranges from 0 to 10, and m ranges from 2 to 11.

2. The CpG oligodeoxynucleotide of claim 1, wherein n ranges from 3 to 4, and q ranges from 1 to 5.

3. The CpG oligodeoxynucleotide of claim 1, wherein
the core sequence is at least one selected from the group consisting of CGCGACGCGTCGCG, ACGATCGAGATCGT, AGGATCGATCCT, TTCGATCGATCGAA, CGATCGATCG, GACGATCGTC, and TGCATCGATGCA;
wherein,
if the core sequence is CGCGACGCGTCGCG, q ranges from 0 to 10, n ranges from 0 to 3, and m ranges from 2 to 11;
or if the core sequence is ACGATCGAGATCGT, q ranges from 0 to 10, n ranges from 2 to 11, and m ranges from 2 to 11;
or if the core sequence is AGGATCGATCCT, q ranges from 0 to 10, n ranges from 2 to 11, and m ranges from 2 to 11;
or if the core sequence is TTCGATCGATCGAA, q ranges from 0 to 10, n ranges from 2 to 11, and m ranges from 2 to 11;
or if the core sequence is CGATCGATCG, q ranges from 0 to 10, n ranges from 1 to 11, and m ranges from 2 to 11;
or if the core sequence is GACGATCGTC, then q is 0, n ranges from 0 to 3, and m ranges from 2 to 11, or then q ranges from 1 to 10, n ranges from 2 to 11, and m ranges from 2 to 11;
or if the core sequence is TGCATCGATGCA, then q is 0, n has a value of 1, 3, 4 or 5, and m ranges from 2 to 11, or then q ranges from 1 to 10, n ranges from 2 to 11, and m ranges from 2 to 11.

4. The CpG oligodeoxynucleotide of claim 1, comprising at least one selected from the group consisting of SEQ ID NO.1 - SEQ ID NO.82.

5. The CpG oligodeoxynucleotide of any one of claims 1-4, wherein the CpG oligodeoxynucleotide comprises at least one selected from the group consisting of: thio-modification, fluorination-modification, methoxy-modification, locked nucleic acid modification, or nanoparticle modification.

6. The CpG oligodeoxynucleotide of claim 5, wherein the thio-modification occurs at the 5' and/or 3' ends of polyguanosine.

7. The CpG oligodeoxynucleotide of claim 5, wherein nanoparticles used in the nanoparticle modification include at least one selected from the group consisting of PLGA, chitosan, lipid nanoparticles, and liposomes.

8. A CpG oligodeoxynucleotide solution formulation, comprising the CpG oligodeoxynucleotide of any one of claims 1 to 7 and excipients, wherein the excipients comprise at least one selected from the group consisting of: pH buffer pair, glycine, trehalose, mannitol, sucrose, arginine, lysine, histidine, glycerol, propylene glycol, Pluronic F127, Pluronic F68, Tween 20, Tween 80, benzalkonium chloride, disodium edetate, sodium citrate, hydroxypropyl methylcellulose, sodium carboxymethyl cellulose, methyl betacyclodextrin, and polyethylene glycol.

9. The formulation of claim 8, wherein metal ions in the CpG oligodeoxynucleotide formulation include sodium, potassium, magnesium, calcium, zinc, and iron, with a total concentration ranging from 0.1 to 90 mM.

10. The formulation of claim 8, wherein the pH of the CpG oligodeoxynucleotide formulation ranges from 7 to 9.

11. Use of the CpG oligodeoxynucleotide of any one of claims 1 to 7 in preparation of drugs for regulating respiratory immune responses.

12. Use of the CpG oligodeoxynucleotide of any one of claims 1 to 7 in preparation of drugs for prevention and treatment of respiratory diseases, wherein the respiratory diseases include at least one selected from the group consisting of respiratory viral infections, respiratory bacterial infections, respiratory fungal infections, respiratory parasitic infections, and respiratory allergic diseases.

13. The use of claim 12, wherein the respiratory viruses include at least one selected from the group consisting of: COVID-19 virus, influenza virus, RSV virus, and SARS-CoV.

14. Use of the CpG oligodeoxynucleotide of any one of claims 1 to 7 in preparation of drugs for prevention and treatment of non-respiratory tract infections, wherein the non-respiratory tract infections include at least one selected from the group consisting of HIV infection, HBV infection, and HCV infection.

15. Use of the CpG oligodeoxynucleotide of any one of claims 1 to 7 in preparation of drugs for regulating proliferation of immune cells or release of immune cell cytokines.

16. Use of the CpG oligodeoxynucleotide of any one of claims 1 to 7 in preparation of immunoadjuvants.

17. Use of the CpG oligodeoxynucleotide of any one of claims 1 to 7 in preparation of drugs for prevention and treatment of viral infections.

18. Use of the CpG oligodeoxynucleotide of any one of claims 1 to 7 in preparation of drugs for treatment of tumors, wherein the CpG oligodeoxynucleotide is the only anti-tumor active ingredient.

19. An anti-tumor composition, comprising the CpG oligodeoxynucleotide of any one of claims 1 to 6 and an anti-tumor drug, wherein the anti-tumor drug includes, but is not limited to, PD-1 anti-tumor drugs, PDL-1 anti-tumor drugs and anti-tumor cell therapy, wherein the CpG oligodeoxynucleotide in the composition serves as a component with anti-tumor efficacy or as an immunoadjuvant.

20. Use of the CpG oligodeoxynucleotide of any one of claims 1 to 7 in preparation of human or animal vaccines.

21. Use of the CpG oligodeoxynucleotide of any one of claims 1 to 7 in treatment of central nervous system diseases, wherein the central nervous system diseases include, but are not limited to, Alzheimer's disease.

22. Use of the CpG oligodeoxynucleotide of any one of claims 1 to 7 in preparation of drugs for prevention and treatment of secondary infections after trauma.

23. Use of the CpG oligodeoxynucleotide of any one of claims 1 to 7 in preparation of drugs for promoting wound healing.

24. Use of the CpG oligodeoxynucleotide of any one of claims 1 to 7 in preparation of drugs for treatment of allergic rhinitis and/or atopic dermatitis and/or asthma and/or chronic obstructive pulmonary diseases and/or eosinophilic-related diseases.

25. A drug prepared from the CpG oligodeoxynucleotide of any one of claims 1 to 7, wherein the drug is in a form of an injection, tablet, lyophilized preparation, inhalant, nasal drops, nasal spray, rectal suppository, eye drops, ointment, lotion, or gel.

26. A CpG oligodeoxynucleotide topical formulation, comprising the CpG oligodeoxynucleotide of any one of claims 1 to 7 and excipients, wherein the excipients include glyceryl behenate, liquid paraffin, petrolatum, medium-chain triglycerides, cocoyl octanoate/decanoate, glyceryl mono- and distearate, hydroxypropyl methylcellulose, carbomer, xanthan gum, sodium carboxymethyl cellulose, poloxamer 407, polyethylene glycol cetostearyl ether, poloxamer 188, oleyl polyoxyethylene glycerol, PEG-7 stearate, Labrasol, sodium lauryl sulfate, propylene glycol monofatty acid ester, polyglyceryl oleate, propylene glycol laurate, propylene glycol, isopropyl myristate, and polyglyceryl oleate.

27. A formulation of a CpG oligodeoxynucleotide adjuvant itself or in combination with other adjuvants for use in human or animal vaccines, comprising the CpG oligodeoxynucleotide of any one of claims 1 to 7 itself, or a combination with aluminum hydroxide adjuvant or aluminum phosphate adjuvant.
